# EUROPEAN PATENT SPECIFICATION

(11) **EP 4 115 921 B1**
(45) Date of publication and mention of the grant of the patent: **10.12.2025**
(21) Application number: 22182471.7
(22) Date of filing: 01.07.2022
(51) Int. Cl.: A61M 5/00, A61J 1/06, A61J 1/14, A61J 1/16, A61J 1/20, A61M 5/32, B65D 1/02, B65D 23/00

(54) **CARTRIDGES, CONTAINERS, AND/OR PROBES, AND SYSTEMS, DEVICES, AND METHODS FOR USING THEM IN TESTING**
KARTUSCHEN, BEHÄLTER, SCHUPPEN ODER SONDEN, UND SYSTEME, VORRICHTUNGEN UND VERFAHREN ZU DEREN VERWENDUNG BEIM TESTEN
CARTOUCHES, RÉCIPIENTS ET/OU SONDES, ET SYSTÈMES, DISPOSITIFS ET PROCÉDÉS POUR LES UTILISER DANS DES TESTS

(30) Priority: 08.07.2021 US 202117305504
(43) Date of publication of application: 11.01.2023
(73) Proprietor: Instrumentation Laboratory Company, Bedford, MA 01730 (US)
(72) Inventor: FARREN, Christopher, Wilmington, 01887 (US); SHREVE, Joshua A., Acton, 01720 (US); FOLSOM, Hugh, Bedford, 01730 (US)
(74) Representative: Müller-Boré & Partner Patentanwälte PartG mbB

(56) References cited:
- WO-A2-2005/044116
- US-A- 3 372 826
- US-B1- 11 054 185
- US-B2- 8 763 829

## Description

The present disclosure relates to a probe assembly, to a testing system comprising a cartridge assembly and a probe system, and to a testing method for accessing a container packaged in a cartridge assembly with a probe system.

US 11 054 185 B1 describes a carrier for supporting containers such as syringes or cartridges in a lyophilization chamber during lyophilization of substances contained therein, wherein the carrier comprises a base assembly with an array of holes for receiving the containers. Each hole includes a heat-conductive metal portion for engagement with lower parts of the container held therein and a relatively nonconductive polymeric portion surrounding a portion of the container above the lower portion for enhancing the vertical growth of relatively large ice crystals. An upper part of the carrier supported above the base assembly includes a plunger-holding plate for frictionally holding elastomeric plungers and a pusher plate movable toward the plunger-holding plate and having protrusions for pushing the plungers out of the plunger-holding plate and into the containers before the containers are removed from the chamber.

WO 2005/044116 A2 describes a cutting device for a blunt needle or transcutaneous sensor for insertion through the derma of a patient, said blunt needle or transcutaneous sensor having a circumference at the distal end, said cutting device comprising a base part and a cutting member for making an incision in the derma, said base part having a track adapted for slideable engagement with the needle or transcutaneous sensor, wherein the cutting member has a cutting width, being less than half the length of the circumference of the blunt needle or transcutaneous sensor.

US 8 763 829 B2 describes a segmented collapsible container for containing a liquid and collapsing when no liquid is present. The container is segmented into a first portion, a second portion, and a third portion. The three segmented portions form a uniform annular sidewall, with each separate portion of the sidewall having a different thickness or material than the other portions. The middle second portion includes a relatively thinner break point in the sidewall to facilitate collapsing. It is this variation in rigidity and integrity of the sidewalls that allows the segmented collapsible container to collapse.

US 3 372 826 A describes a composite container for packaging a product, the container being comprised of a paste molded glass envelope of substantially thin, uniform wall thickness and distribution. The glass envelope is seamless and has a lower bulbous portion that is substantially spherically contoured which defines the major diameter of the envelope. The upper portion of the envelope terminates in an orifice defining a mouth or finish of the container which receives a closure. A fitment made of a yieldable, less frangible material comprises a cup-shaped bottom or base that is adhesively or otherwise secured to the lower bulbous portion of the glass envelope.

### BRIEF DESCRIPTION OF THE DRAWINGS

The accompanying drawings, together with the description, serve to explain the disclosed embodiments. In the drawings:
FIG. 1A is an illustration of a cartridge assembly;
FIG. 1B is an illustration of a top view of the cartridge assembly of FIG. 1A;
FIG. 2 is an illustration of a cartridge assembly;
FIG. 3 is an illustration of a cartridge assembly accommodating a container;
FIG. 4A is an illustration of a cartridge assembly accommodating a container;
FIG. 4B is another illustration of the cartridge assembly of FIG. 4A;
FIG. 5A is an illustration of a probe system;
FIG. 5B is an illustration of a tip of an inner needle;
FIG. 6A is an illustration of a needle;
FIG. 6B is an illustration of another needle;
FIG. 6C is an illustration of another needle;
FIG. 7 is an illustration of a probe system for piercing a stopper of a container;
FIG. 8 is another illustration of a probe system for piercing a stopper of a container;
FIG. 9 is an illustration of a pierceable stopper for a container;
FIG. 10 is an illustration of a comparison of stopper fragmentation in two different stoppers;
FIG. 11A is an illustration of a container;
FIG. 11B is an illustration of another container;
FIG. 12 is an illustration of a comparison of two different containers;
FIG. 13 is an illustration of a dead volume comparison for two different containers;
FIG. 14A is an illustration of another container; and
FIG. 14B is an illustration of another container.

### DETAILED DESCRIPTION

Exemplary embodiments are described with reference to the accompanying drawings. While examples and features of disclosed principles are described herein, modifications, adaptations, and other implementations are possible . Exemplary embodiments described herein may be independent of each other. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It should also be noted that as used herein and in the appended claims, the singular forms "a," "an," and "the" include plural references unless the context clearly dictates otherwise. For convenience, the term "disclosed embodiments" or "exemplary embodiment" may be used herein to refer to a single embodiment or multiple embodiments of the disclosure.

Fluids, such as reagent materials, are often packaged and stored in vials. Multiple vials can then be packaged and stored in a cartridge format to improve usability. Such cartridge-based reagents often contain multiple forms of packaging within the cartridge system. For example, the reagents may be directly packaged in a primary container (e.g., a vial having a stopper). The primary container may be contained within a secondary container (e.g., a plastic housing or cartridge). Accordingly, an automated probe system may interface with the primary or secondary container during use (e.g., to aspirate material from the primary container).

Additionally, an automated probe system may perform multiple tests using reagents stored in the same primary containers, requiring multiple aspirations from these primary containers. Multiple aspirations may require repeated penetrations of stoppers sealing the primary containers. But repeated penetrations of conventional stoppers can reduce the reliability of automated probe systems using such stoppers. Repeated penetrations of conventional stoppers can cause stopper coring and fragmentation, which can interfere with reagent aspiration, and can expose reagent materials in the primary container to the ambient environment (e.g., through air holes in the stopper), which can reduce the lifetime, following the initial penetration of the stopper, of the reagent materials stored inside the primary container (e.g., the onboard stability of the reagent materials).

Additionally, automated probe systems can exhibit reagent wastage when used with conventional primary containers. Such systems may aspirate reagents using a probe advanced into the primary containers. The probe tip may not contact the bottom of the primary containers, to avoid damaging or blocking the probe (or the containers). Accordingly, the probe may be unable to aspirate reagents remaining at the bottom of the primary containers, below the probe tip. The remaining reagent, often referred to as "dead volume," may be considered lost material and may be thrown out.

Additionally, automated probe systems can be used with a broad range of primary container stoppers. Such stoppers may be composed of various materials and have varying dimensions. However, conventional automated probe systems accommodate a single piercing probe and may therefore be unable to reliably pierce various types of stoppered containers.

Additionally, conventional combinations of primary and secondary containers may use space inefficiently. For example, a rectangular cartridge may store cylindrical reagent vials, resulting in some volume of the rectangular cartridge being unused or wasted.

The disclosed cartridges, containers, and/or probes (and systems, methods, and devices of using such cartridges, containers, and/or probes), independently or in combination, can improve upon conventional designs as follows: An improved cartridge assembly can allow an automated probe system to access the containers packaged therein without removing the containers from the cartridge assembly. An improved cartridge assembly can accommodate variously sized and shaped containers so as to minimize unused cartridge assembly volume. An improved cartridge assembly can align the cartridge assembly and the containers stored therein with the automated probe system to ensure accurate penetrations of the stoppers and accurate aspirations. The repeated penetration can be accurately at the same location of the stopper to reduce stopper fragmentation. An improved primary container stopper can support repeated probe penetration with reduced stopper coring and fragmentation. The improved stopper can self-seal after probe withdrawal, improving preservation and extending the onboard stability of the reagents stored in the primary container (e.g., from about 2 weeks to about 4 weeks). The stopper may self-seal by ensuring that the probe pierces the same exact location on the stopper each time the probe penetrates the stopper. By driving the probe to find the initial piercing location and preventing creating multiple piercing spots on the stopper, the stopper may be able to self-seal even after multiple penetrations. An improved container for storing reagents can be shaped to minimize dead volume. An improved probe system can include a multi-mode probe suitable for piercing primary container stoppers of various sizes, materials, and/or types.

The present disclosure relates generally to cartridge assemblies, containers, and/or probes suitable for use in testing, e.g., diagnostic testing including but not limited to medical (disease/drug), chemical, and/or biological testing/analysis, and systems, devices, and methods of using such cartridges, containers, and/or probes in such testing.

A probe assembly, a testing system including a cartridge assembly and a probe system, and a testing method for accessing a container packaged in a cartridge assembly with a probe system are defined in the appended claims.

As discussed in further detail below, various cartridge assemblies for accurate positioning and access of containers and components stored therein are provided. The cartridge assemblies can support alignment of an automated probe system, conventional or as described in the present disclosure, with assemblies and containers packaged within the cartridge assemblies, where such assemblies and containers can be conventional or have one or more features described in the present disclosure. Accordingly, the disclosed cartridge assemblies can support accurate positioning of an automated probe system. which may reduce stopper fragmentation when the probe punctures the stoppers of the containers. For example, when a probe, such as a probe with a rounded tip, punctures a stopper at a location where the stopper was punctured previously, there is a reduction in friction and a reduction in the tearing of the stopper, thereby minimizing fragmentation. By positionally aligning the probe to the cartridge assembly and accessing the contents within containers, tolerance stacking issues that could lead to inaccuracy and repeatability issues may be eliminated. Moreover, the rounded tip of the probe may also allow the probe to locate the location on the stopper that has previously been pierced without a sharp edge digging into the stopper. The rounded tip may also guide the probe into the previously pierced location, thereby reducing stopper fragmentation. In some embodiments, independent of the alignment functionalities, the cartridge assemblies may comprise cavities capable of accommodating various types, sizes, and/or diameters of containers. For example, the cartridge assemblies may be configured to interchangeably accommodate cylindrical containers and oblong containers, or containers of other shapes.

Additionally, various embodiments of a probe system, such as an automated probe system, for penetrating a stopper of a container are provided, the stopper or container being conventional or including features of the present disclosure. The probe system, consistent with the embodiments of the present disclosure, includes a dual-mode probe system having an inner probe and an outer probe. The inner probe and the outer probe of the probe system are independently actuatable. Whether the inner probe or the outer probe penetrates the stopper can depend on a type of the container. In some implementations, the outer probe can be used to penetrate containers including relatively thicker stoppers, or containers intended to be penetrated relatively few times (e.g., blood sample containers, or other sample containers). The inner probe can be used to penetrate containers having relatively thinner stoppers, or containers intended to be penetrated many times (e.g., reagent containers, or the like). As used herein, the term "probe" may be used interchangeably with the term "needle". The term "probe" may refer to the probe system, or a specific component therein such as a needle. For example, the term "probe" may refer to a needle comprising a thin, hollow metal tube through which fluid can be aspirated and dispensed. Accordingly, the term "probe" may refer generally to a needle or any elongated component comprising a hollow cavity that is capable of puncturing, piercing, and penetrating a structure and aspirating material through the hollow cavity. According to another embodiment of the present disclosure, the inner probe can be used to penetrate containers including relatively thicker stoppers, or containers intended to be penetrated relatively few times, and the outer probe can be used to penetrate containers having relatively thinner stoppers, or containers intended to be penetrated many times.

Various embodiments of a stopper for a container are provided. The stopper, consistent with the embodiments of the present disclosure, may be configured to reduce and/or minimize stopper fragmentation when repeatedly penetrated by a probe. The stopper can be configured to seal, and self-seal after being penetrated, a container or vial for storing lyophilized materials. In some embodiments, the stopper may be made of a halogenated butyl rubber material such as bromobutyl such that the stopper can self-seal after penetration. Additionally or alternatively, the stopper, consistent with the embodiments of the present disclosure, may include a bulb-shaped internal cross-section, which may allow the stopper to be repeatedly penetrated without creating substantial stopper fragmentation.

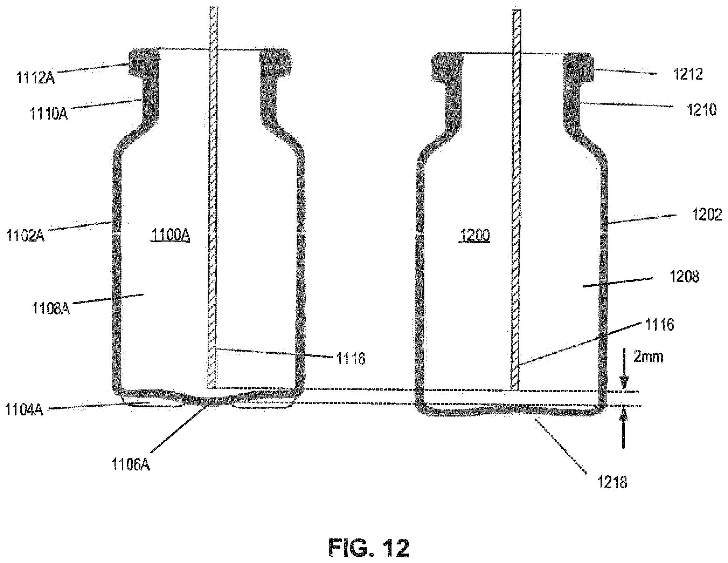

Moreover, various embodiments of a container for storing and packaging materials, such as reagent materials, are provided. The container, consistent with the embodiments of the present disclosure, may be configured to reduce the dead volume of material remaining at the bottom of the container below the furthest reach of a probe. In some embodiments, for example, the container may be shaped to pool material on the bottom surface of the container (e.g., pooling material toward the center of the interior lower surface), thereby enabling a probe to aspirate more of the material in the container and reducing dead volume as compared to a conventional base design (e.g., as in the right image depicted in FIG. 12).

The present disclosure relates generally to cartridges, containers, and/or probes, and systems, devices, and methods of using such cartridges, containers, and/or probes in testing, e.g., diagnostic testing. A container for storing materials has a stopper and, more particularly, a pierceable lyophilization stopper that is capable of being repeatedly penetrated using an aspiration needle without stopper fragmentation. The stopper may self-seal after each penetration, extending the onboard stability of the materials in the container. The present disclosure also relates to a probe assembly for piercing a container as defined in the appended claims and, more particularly, to a dual-mode probe assembly for piercing a container and for aspiring materials from the container. Each mode of the probe can be used for a different purpose, e.g., piercing and aspiring different containers or materials. At least one mode of the probe is designed such that the probe in this mode can repeatedly pierce a container without causing excessive fragmentation of the stopper of the container or preventing self-sealing of the stopper. Additionally, the present disclosure relates generally to a container and, more particularly, to a container with a bottom surface that is configured to pool fluid in a center. The pooling can help aspiration of the material from the container and can reduce material waste. The present disclosure further relates to a cartridge assembly and, more particularly, to a cartridge assembly that is capable of automatically aligning the cartridge assembly and a container accommodated therein with a probe assembly. The automation can increase test throughput, reduce human incurred error, and reduce human burdens on carrying out the tests.

Although the present disclosure describes, e.g., containers, probes, cartridges, etc. that can be implemented in a system altogether, each can be used independently with systems and devices that are not described in this disclosure, e.g., conventional systems and devices. For example, the stoppers of the containers having the stoppers described herein can be used in a system or device that do not implement any of the probes, cartridges, or container bottom designs as described herein. Likewise, the probes, cartridge systems, and the containers with the bottom surface as described herein can each be independently used without regard to each other. The benefits provided by each one is independent of implementing the other one in the same system. The present disclosure merely describes the various aspects in a related manner for convenience.

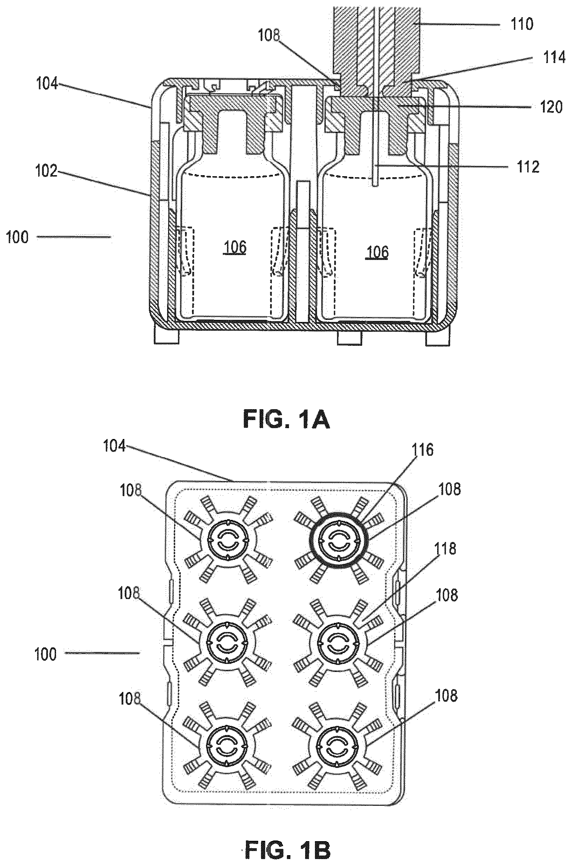

Turning now to the drawings, FIGS. 1A and 1B illustrate an exemplary cartridge assembly 100 for storing and packaging one or more containers 106. In the illustrated embodiment, cartridge assembly 100 comprises a first housing 102 and a second housing 104 configured to be coupled to first housing 102. In some embodiments, the first housing 102 and the second housing 104 may be coupled to each other by a latch or snap-fit connection. In other embodiments, the first housing 102 and the second housing 104 may be adhered, joined, or welded to each other so as to form a single housing. The disclosed embodiments are not limited to a particular method of coupling the first housing 102 and the second housing 104. As discussed below, the first housing 102 comprises a first set of cavities configured to accommodate one or more containers 106. One or more containers 106 may be filled with a material, such as a reagent material, and are stoppered by a stopper. For example, one or more containers 106 may comprise vials that are filled with reagents and stoppered. Additionally second housing 104 comprises a second set of cavities that correspond to the first set of cavities when second housing 104 is coupled to first housing 102. In some embodiments, first housing 102 and second housing 104 may be manufactured using plastic, such as multi-component plastic. In some embodiments, first housing 102 and second housing 104 can be formed as separate components (which can be affixed after containers 106 are inserted in the cavities). In various embodiments, first housing 102 and second housing 104 can be formed connected by an integrated hinge (e.g., a living hinge). In embodiments, the cartridge assembly includes, for each cavity of the second housing, means for aligning the cartridge assembly and a container accommodated in the cavity with a probe assembly. As described herein, such means include one or more of, for each cavity, means for aligning the cartridge assembly with the proximal end of probe assembly, and can include means for securing the container within the cartridge assembly, or means for aligning the container accommodated with the cavity.

### Cartridge-probe alignment

As illustrated in FIG. 1A, second housing 104 may comprise means for aligning the cartridge assembly with probe assembly 110. In some embodiments, second housing 104 can comprise a plurality of alignment features 108 configured to align cartridge assembly 100 with a probe assembly 110. The alignment features 108 may correspond to the first set of cavities and/or the second set of cavities. For example, each of the alignment features 108 may be positioned coaxial to a center of a corresponding cavity of the first set of cavities and/or the second set of cavities. The alignment features 108 may be configured to mate with a component 114 at a proximal end of the probe assembly 110. In some embodiments, as illustrated in FIG. 1B for example, the alignment features 108 may comprise a plurality of openings, such as circular openings 116 on a top surface of second housing 104. In some embodiments, the plurality of openings may comprise different shapes, such as rectangular openings, triangular openings, or any other polygonal openings. Component 114 may comprise a protrusion, such as a cylindrical protrusion, at a proximal end of probe assembly 110. By way of example, when the alignment features 108 mate with component 114 of probe assembly 110, the openings 116 of the alignment features 108 may be configured to accommodate component 114 protruding at the proximal end of probe assembly 110. For example, the alignment features 108 and/or component 114 of probe assembly 110 may comprise one or more tapers to compensate for misalignments between the probe assembly 110 and the cartridge assembly 100. In some embodiments, on a bottom surface of component 114, relief cutouts may be provided and positioned to correspond to a plurality of spring features 118 provided on a top surface of second housing. The spring features 118 may push down on one or more containers 106 to prevent the containers 106 from moving around in the cartridge assembly 100. In addition, the bottom surface of component 114 may comprise a flat surface configured to contact the top surface of the containers 106 and to hold down the containers 106 vertically so as to keep the containers 106 stable. Accordingly, when the alignment features 108 mate with component 114 of probe assembly 110, a probe 112 of probe assembly 110 may be automatically and accurately aligned with a corresponding container 106 and may be positioned coaxial to a center of the corresponding container 106. As such, when probe 112 is actuated to penetrate a stopper 120 of the corresponding container 106, probe 112 may penetrate a center of the stopper 120. In this manner, alignment features 108 and component 114 can interact to reduce targeting precision errors that might otherwise arise from an accumulation of tolerances (e.g., misalignment tolerances between probe assembly 110 and the center of the cavity corresponding to alignment features 108, misalignment tolerances between a center of an opening in the container and the center of the cavity, or the like).

Accuracy and precision in penetrating the stopper 120 may reduce stopper fragmentation. Accurate alignment such that probe 112 penetrates a center of stopper 120 instead of a side wall of stopper 120 may be important in reducing stopper fragmentation, as repeatedly piercing a side wall of stopper 120 may cause stopper fragmentation. Precise alignment may increase the likelihood that each penetration of stopper 120 by probe 112 occurs at the same location (e.g., through the same hole) on the stopper 120. When multiple penetrations occur in close proximity (but not at same location), fragments can separate from the stopper 120. Such fragments may interfere with aspiration of reagents. In some instances, separation of a fragment may cause a non-sealing hole to form in stopper 120, exposing reagents to the environment. Thus accurate and precise probe targeting can reduce fragmentation of stopper 120 and improve the onboard stability of reagents stored in the container 106.

In some embodiments, second housing 104 may further comprise means for securing one or more containers 106 within the cartridge assembly when the second housing is coupled to the first housing. In some embodiments, the securing means can be configured to apply a force vertically downward on one or more containers 106 accommodated within cartridge assembly 100. In some embodiments, the applied force may be reduced when a container (or an opening of the container) is aligned coaxially with openings on the top surface of second housing 104. Thus, the securing means can both secure the one or more containers 106 within the cartridge assembly 100 can align the one or more containers 106 with the openings (and thus with the proximal end of probe assembly 110).

In some embodiments, the securing means can include a plurality of protrusions, such as the plurality of spring features 118, positioned circumferentially around each of the plurality of opening of alignment features 108. The spring features 118, for example, may be biased vertically downward and may be configured to apply a force vertically downward. Accordingly, when one or more containers 106 are accommodated in cartridge assembly 100, the plurality of spring features 118 positioned circumferentially around the alignment features 108 may be configured to apply a force vertically downward on top of the one or more containers 106. In this manner, the plurality of spring features 118 can secure the one or more containers 106 inside cartridge assembly 100. In some embodiments, the plurality of spring features 118 may be manufactured using plastic and may be flexible. In various embodiments, the securing means can include compressible foam, plastic, rubber, metal, or other materials. Such materials can be positioned between the one or more containers 106 and the second housing when the second housing 104 is coupled to the first housing 102. The materials can be attached to the second housing 104 or to disposed around the neck of the one or more containers 106. When the second housing 104 is coupled to the first housing, the materials can be compressed and therefore provide aligning forces and force vertically downward onto the one or more containers 106.

Additionally, or alternatively, the first housing 102 may comprise a plurality of aligning means, similar to spring features 118, that are configured to apply force inward to the sidewalls of one or more containers 106 accommodated within the cartridge assembly 100. In some embodiments, the applied force may be reduced when a container (or an opening of the container) is aligned the cavity (or with an opening of the cartridge assembly). Thus, the aligning means can secure the one or more containers 106 in a desired position within the cartridge assembly 100. In some embodiments, the container (or opening of the container) can be coaxially aligned with the cavity (or with an opening of the cartridge assembly).

As explained previously, none of the alignment features 108, spring features 118, or circular openings 116 of the cartridge assembly is limited to any other aspects, e.g., the probe configurations, the stopper configurations, or the primary container configurations of the present disclosure. The cartridge-probe alignment features described above can be applied to any conventional probes, stoppers, or primary containers.

### Cartridge assembly configurations

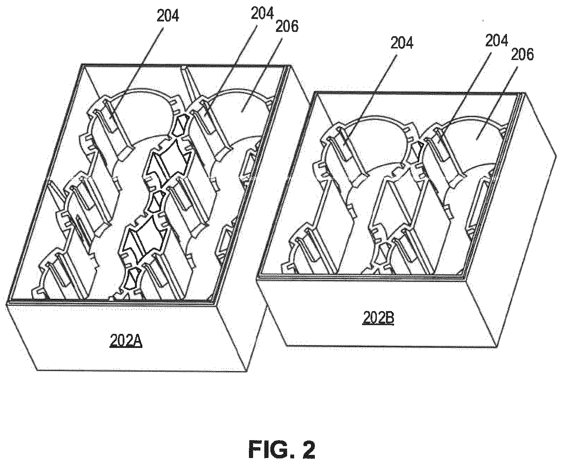

Referring now to FIG. 2, various embodiments of a cartridge assembly, such as cartridge assembly 100 of FIGS. 1A and 1B, are illustrated. As illustrated in FIG. 2, first housings of cartridge assemblies 202A, 202B comprise a plurality of cavities 206 configured to accommodate one or more containers, such as containers 106 of FIG. 1A. For example, a first housing of cartridge assembly 202A may comprise six cavities 206, and a first housing of cartridge assembly 202B may comprise four cavities 206. Accordingly, cartridge assembly 202A may be configured to accommodate a number of containers that is different from a number of containers that cartridge assembly 202B may be configured to accommodate. In some embodiments, the cavities 206 can be interconnected (for example, three cavities 206 are connected in cartridge assembly 202A, and two cavities 206 are connected in cartridge assembly 202B) such that containers of different sizes can fit into one cavity 206, or two or three interconnected cavities 206. Additionally, or alternatively, cartridge assemblies may comprise four or more interconnected cavities 206. With the various numbers of cavities 206, cartridge assemblies 202A, 202B may provide modularity and flexibility to mix and match various numbers, shapes, and/or sizes of containers, thereby allowing the use of larger containers inside cartridge assemblies interchangeably with smaller containers.

In some embodiments, a spring system, e.g., one integrated with the cartridge assembly, can be configured to apply force to sidewalls of one or more containers packaged therein. In this manner, the integrated spring system can secure the one or more containers in a desired position within the cartridge assemblies. By way of example, as illustrated in FIG. 2, cartridge assemblies 202A, 202B may comprise a plurality of protrusions 204 protruding inward from the sidewalls of the cavities 206. The plurality of protrusions 204 may be flexible and may be manufactured using plastic or metal. The plurality of protrusions 204 may be biased inward. When a container is positioned within a cavity, the container may outwardly displace the protrusions in the cavity. Thus, the plurality of protrusions 204 may be configured to apply force to sidewalls of one or more containers. Accordingly, the force applied by the plurality of protrusions 204 may secure the one or more containers accommodated in cartridge assemblies 202A, 202B and position the one or more containers in desired locations within cartridge assemblies 202A, 202B. For example, the force applied may coaxially align the one or more containers accommodated in the cavities 206 (or openings in the tops of said one or more containers) with corresponding centers of the cavities 206. Additionally, the force applied by the plurality of protrusions 204 may be configured to hold the one or more containers rigidly in place and prevent or reduce movement of the one or more containers within cartridge assemblies 202A, 202B. Therefore, even when cartridge assemblies 202A, 202B are translationally and laterally shifted during transfer, the plurality of protrusions 204 may hold the one or more containers in place and maintain accurate alignment of a probe assembly, such as probe assembly 110 of FIG. 1A, with the one or more containers when cartridge assemblies 202A, 202B mate with the probe assembly. Accurate alignment of the probe assembly with the one or more containers in cartridge assemblies 202A, 202B may reduce tolerance stack-up issues, allow for precise targeting of the probe assembly, increase reliability of the probe assembly (e.g., by extending on-board stability and reducing stopper fragmentation). Accurate alignment between the probe assembly and the cartridge assembly such that the probe pierces a center (instead of the side walls) of the one or more containers in the cartridge assembly may be important in minimizing stopper fragmentation, as repeatedly piercing a side wall of a stopper may cause excessive stopper fragmentation. Precise alignment can increase the likelihood of each penetration occurring at the same location in the stopper (e.g., through the same hole). Thus accurate and precise penetration can extend on-board stability and reduce stopper fragmentation.

In some embodiments, each cavity 206 may comprise an insert manufactured separately from the cartridge assembly and affixed to the first housing of the cartridge assembly. The inserts may be manufactured using, for example, plastic, metal, compressible foam, rubber, or other flexible materials that could apply a force horizontally or inward onto the side walls of one or more containers stored therein. Accordingly, the insert may align the one or more containers to a probe assembly.

In various embodiments, the aligning means can include compressible foam, plastic, rubber, metal, or other materials. Such materials can be positioned between the one or more containers 106 and the sidewalls of the cavities within the cartridge assemblies. The materials can be attached to the second housing or to disposed around the one or more containers 106. When the second housing is coupled to the first housing, the materials can be compressed and therefore provide aligning forces onto the sidewalls of the one or more containers 106.

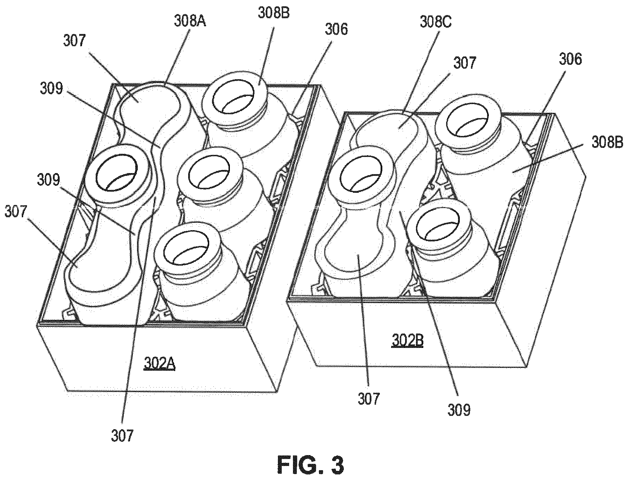

FIG. 3 illustrates a cartridge assembly accommodating various containers. As illustrated in FIG. 3, a first housing of cartridge assembly 302A may be similar to the first housing of cartridge assembly 202A of FIG. 2 and may comprise six cavities 306 similar to the six cavities 206 of cartridge assembly 202A. Each three adjacent cavities are interconnected. Additionally, a first housing of cartridge assembly 302B may be similar to the first housing of cartridge assembly 202B of FIG. 2 and may comprise four cavities 306 similar to the four cavities 206 of cartridge assembly 202B. Each two adjacent cavities are interconnected. As discussed above, cartridge assemblies 302A, 302B may be configured to accommodate various types, sizes, and diameters of containers.

As illustrated in FIG. 3, cartridge assembly 302A may be configured to accommodate in cavities 306 containers of varying shapes and dimensions. In this example, container 308A may be oblong in shape and container 308B may be cylindrical in shape. Cartridge assembly 302B may likewise be configured to accommodate in cavities 306 containers of varying dimensions, such as containers 308B and container 308C. In some embodiments, container 308A may comprise a volume between 15 ml and 200 ml, containers 308B may each comprise a volume between 4 ml and 60 ml, and container 308C may comprise a volume between 10 ml and 150 ml. As illustrated in FIG. 3, containers 308B may comprise single containers with a single lobe 307, container 308C may comprise two lobes 307 connected to each other by an indentation 309, and container 308A may comprise three lobes 307 connected to each other by indentations 309. Each cavity 306 in cartridge assemblies 302A, 302B may be shaped and configured to accommodate a single lobe 307 of a container. Accordingly, container 308A having three lobes 307 may be accommodated in three adjacent cavities 306, and container 308C having two lobes 307 may be accommodated in two adjacent cavities 306.

While FIG. 3 illustrates cartridge assembly 302A accommodating one container 308A and three containers 308B and cartridge assembly 302B accommodating one container 308C and two containers 308B, the embodiments of the present disclosure are not limited to the configurations illustrated in FIG. 3. For example, cartridge assembly 302A may accommodate (i) six containers 308B, (ii) one container 308C and four containers 308B, (iii) two containers 308C and two containers 308B, (iv) one container 308C, one container 308B, and one container 308A, or (v) two containers 308A. Additionally, or alternatively, cartridge assembly 302B may accommodate (i) four containers 308B or (ii) two containers 308C. In other embodiments, not all of the cavities 306 may need to accommodate a container.

As shown in FIG. 3, cartridge assemblies 302A, 302B may allow various types and sizes of containers 308A, 308B, 308C to be interchangeably packaged therein, thereby maximizing configuration flexibility and increasing packaging efficiency. For example, cartridge assemblies 302A, 302B provide modularity and flexibility to mix and match various shapes and sizes of containers and, thus, allow the use of larger containers, such as containers 308A, 308C, inside cartridge assemblies interchangeably with smaller containers, such as container 308B. Accordingly, if a particular test requires more of a first material and less of a second material, the first material can be stored in a larger container, such as containers 308A, 308C, while the second material can be stored in a smaller container, such as container 308B. Similarly, if a particular assay requires different amounts of different components, larger containers, such as containers 308A, 308C, can be used to store larger amounts of a particular component and smaller containers, such as container 308B, can be used to store smaller amounts of another component, thereby providing a cost-effective cartridge assembly, making efficient use of space within a cartridge assembly, and allowing more materials to be packaged in the same amount of space within a cartridge assembly.

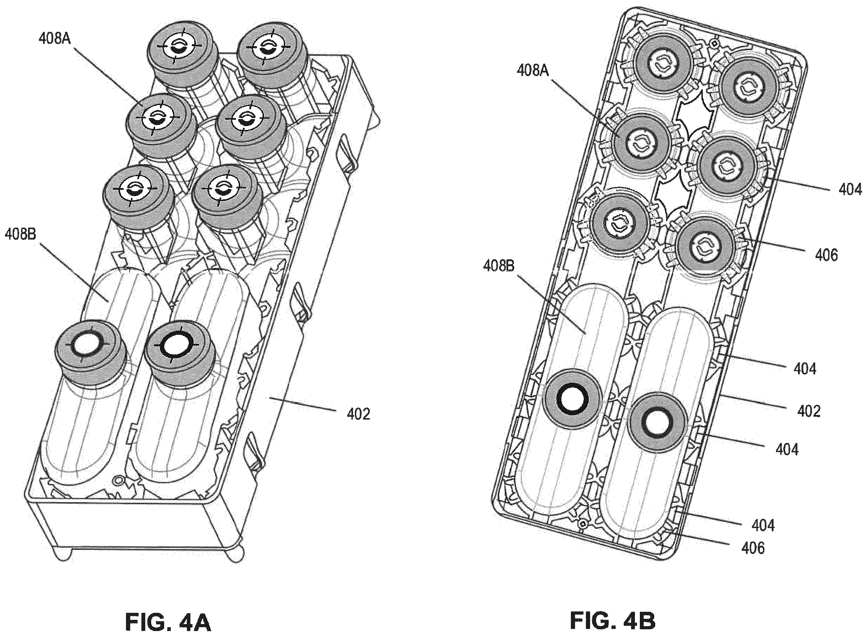

In some embodiments, cartridge assemblies may comprise between 2 and 20 cavities configured to accommodate containers. FIGS. 4A and 4B illustrate a cartridge assembly 402. By way of example, cartridge assembly 402 may comprise 12 cavities 404 configured to accommodate various types and sizes of containers. As shown in FIGS. 4A and 4B, cartridge assembly 402 may comprise two oblong containers 408B and six single containers 408A that are cylindrical. In some embodiments, the oblong containers 408B may not include any indentations. The oblong containers 408B may therefore possess straight sidewalls. Each of the two oblong containers 408B may be shaped and configured to fit the cavities 404 of cartridge assembly 402. For example, cavities 404 may be manufactured using injection molded plastic and may be configured to stretch to accommodate an oblong container 408B. In some embodiments, the housing of cartridge assembly 402 may comprise a plurality of protrusions 406 protruding inward from the sidewalls of each cavity 404. The plurality of protrusions 406 may be biased inward. Accordingly, when oblong containers 408B and/or single containers 408A are accommodated within the cavities 404, the plurality of protrusions 406 may be configured to apply force to the sidewalls of the containers 408A, 408B, so as to secure the containers 408A, 408B and hold the containers 408A, 408B in a desired location within cartridge assembly 402.

### Probes

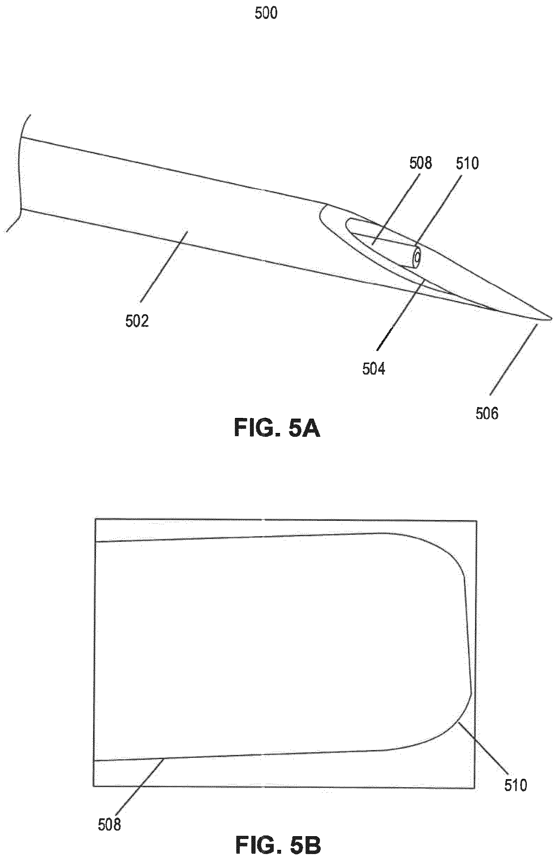

Referring now to FIGS. 5A and 5B, a probe system 500 is shown for piercing a container, such as containers 106 of FIG. 1A, containers 308A, 308B, 308C of FIG. 3, and containers 408A, 408B of FIGS. 4A and 5B, or any other conventional or commercially available containers. Probe system 500 comprises an outer needle 502 comprising a hollow cavity 504 and a pointed tip 506 at a proximal end thereof, and an inner needle 508 disposed inside the hollow cavity 504 of the outer needle 502. The inner needle 508 comprises a rounded tip 510 at a proximal end thereof.

As illustrated in FIG. 5A, the outer needle 502 and the inner needle 508 are positioned coaxial relative to each other. The outer needle 502 and the inner needle 508 are independently actuatable to pierce various types of containers that are stoppered. By way of example, the outer needle 502 is configured to extend from a first position to a second position when actuated, and the inner needle 508 is configured to extend from a third position to a fourth position when actuated. When actuated, one of the outer needle 502 and the inner needle 508 is configured to move to puncture a stopper of a container. In some embodiments, the outer needle 502 and the inner needle 508 may be manufactured using metal, such as stainless steel or nickel-cobalt base alloy (such as MP35N^{®}). In some embodiments, the outer needle 502 and the inner needle 508 may be manufactured using the same material. In other embodiments, the outer needle 502 and the inner needle 508 may be manufactured using different material.

In some embodiments, the rounded tip 510 of the inner needle 508 may reduce stopper fragmentation when the inner needle 508 repeatedly punctures a stopper of a container. For example, the rounded tip 510 may guide the inner needle 508 into a previously pierced location on the stopper, thereby reducing stopper fragmentation. Additionally, when the inner needle 508 punctures a stopper at a location where the stopper was punctured previously, there may be a reduction in friction and therefore a reduction in the tearing of the stopper, thereby reducing fragmentation. Moreover, the rounded tip 510 of the inner needle 508 may also allow the inner needle 508 to locate the location on the stopper that has previously been pierced without a sharp edge digging into the stopper.

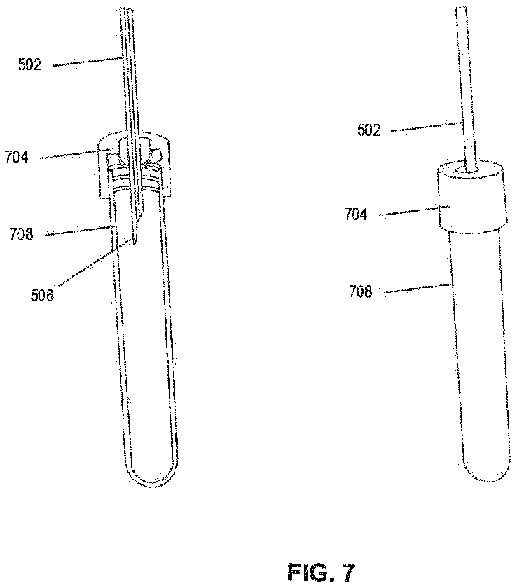

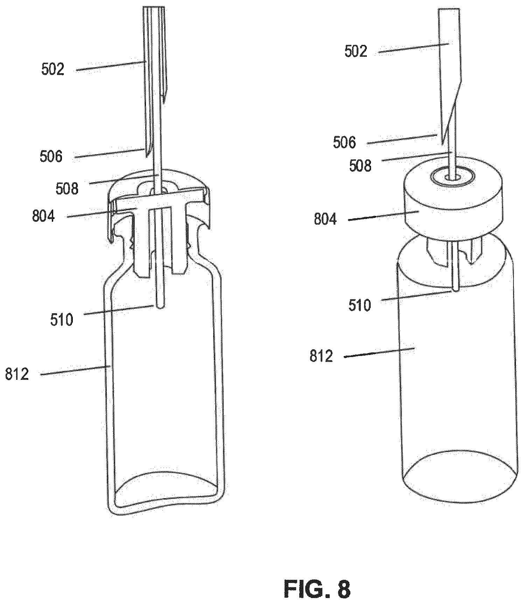

The probe system 500 comprises a dual-mode piercing probe for piercing a stopper of a container. Accordingly, one of the outer needle 502 and the inner needle 508 may be actuated to puncture a stopper of a container based on the type of container and/or the type of stopper. By way of example, as illustrated in FIG. 7, when the container is a container 708 having a thick stopper 704, the outer needle 502 with the pointed tip 506 may be configured to move to puncture the stopper 704 when actuated. The sharp pointed tip 506 can be effective in piercing a thick stopper like the stopper 704. The container 708 can be a sample container that contains samples, e.g., blood samples, for testing. Commercially available containers like container 708 include blood sample tubes manufactured by Becton Dickinson and Greiner. In some implementations, a stopper of such containers can have a thickness of about 1 mm to about 10 mm. Alternatively, as illustrated in FIG. 8, when the container is a container 812 having a thin stopper 804, the inner needle 508 with a rounded tip 510 may be configured to move to puncture the stopper 804 when actuated. The container 812 can be commercially available containers include containers or vials manufactured by OMPI, Schott, and Wheaton, or the containers described herein. The container 812 can be a reagent container. In some implementations, a stopper of such containers 812 can have a thickness of about 1 mm to about 5 mm. The relatively thin stopper can allow the use of the rounded tip 510 for piercing the stopper. Accordingly, the probe system 500 may be used successfully puncture and access various containers with various stoppers. In addition, because some needles are better suited to puncture specific types of stoppers, the probe system 500 may be capable of selecting the appropriate needle to puncture a stopper of a container, depending on the type of container and/or type of stopper, thereby reducing friction between the needle and the stopper during repeated penetration of the stopper, reducing stopper fragmentation, and improving system reliability. One or more of the stopper design, the probe tip design, elimination of sharp edges, and cartridge/probe alignment may facilitate precise targeting between the probe and the stopper of the container, and thus, reduce stopper fragmentation by ensuring that the probe punctures the stopper at the same exact location each time. In some implementations, the system implementing the probe system 500 can be configured to automatically control the probe system 500. Such automatic control can include automatic detection of the type of containers or stoppers to penetrate (or being penetrated), and automatic selection of the proper mode or needle for penetrating such containers or stoppers. In some implementations, probe system 500 can include at least one processor and a memory containing instructions that, when executed by the at least one processor, cause probe system 500 to detect the type of containers or stoppers, select the proper mode or needle, and perform the aspiration.

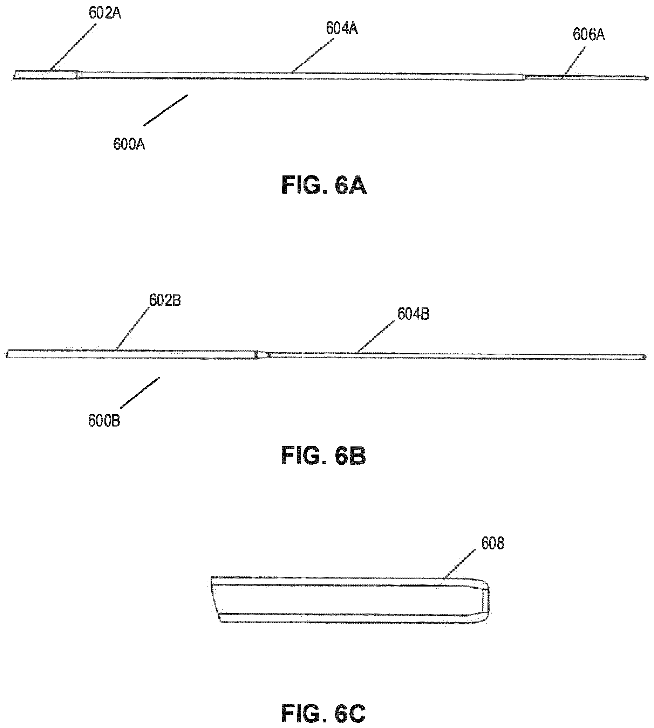

FIGS. 6A-6C illustrate a needle, such as inner needle 508 of FIGS. 5A and 5B. As illustrated in FIGS. 6A and 6B, a needle of a probe system (e.g., probe system 500 of FIGS. 5A and 5B) may comprise a plurality of stepped sections having different diameters. For example, needle 600A may comprise three stepped sections 602A, 604A, 606A (as shown in FIG. 6A), or needle 600B may comprise two stepped sections 602B, 604B (as shown in FIG. 6B). The diameter of the sections increases from the distal end section (e.g., the section that first penetrates a stopper) to the near end section, while each section can have a constant diameter. The small diameter can facilitate stopper penetration, while the large diameter can provide sufficient internal volume in the needle to support application of the needle to the stopper. Although needles with two or three sections are shown, a needle can have more than three sections, e.g., four, five, or more. In other embodiments, a needle may be tapered that continuously increase a diameter of the needle without any stepped sections. In this example shown in the figures, section 602A may have a larger diameter than section 604A, and section 604A may have a larger diameter than section 606A. Similarly, section 602B may have a larger diameter than section 604B. In other embodiments, the needle may comprise more than three stepped sections, such as between four and ten stepped sections. In some embodiments, section 606A and at least a portion of section 604A of needle 600A may be configured to penetrate a stopper and extend into a stoppered container when actuated. The diameter of section 604A may be greater than the diameter of section 606A so as to provide sufficient internal volume in the needle 600A to support aspiration of sufficient volumes for testing applications. Even though the diameter of section 604A is greater than the diameter of section 606A, the piercing force of needle 600A when penetrating the stopper may not increase stopper fragmentation because section 606A with a smaller diameter may initially pierce and pass through the stopper. In other embodiments, section 604B of needle 600B may be configured to penetrate a stopper and extend into a stoppered container when actuated. Even though the diameter of section 602B is greater than the diameter of section 604B, the piercing force of needle 600B when penetrating the stopper may not increase stopper fragmentation because section 604B with a smaller diameter may initially pierce and pass through the stopper. In some embodiments, needles 600A, 600B may be manufactured using metal, such as stainless steel or nickel-cobalt base alloy (MP35N).

Additionally, or alternatively, as illustrated in FIG. 6C, needles 600A, 600B may comprise a rounded tip 608 and a nozzle-shaped interior cross-section. In some embodiments, the rounded tip 608 may facilitate accurate positioning of needles 600A, 600B. which may reduce stopper fragmentation when needles 600A, 600B penetrate a stopper of a container. For example, when needles 600A, 600B with rounded tip 608 penetrate a stopper at a location where the stopper was penetrated previously, there is a reduction in friction and a reduction in the tearing of the stopper, thereby minimizing fragmentation. Moreover, the rounded tip 608 may also allow the needles 600A, 600B to locate the location on the stopper that has previously been pierced without a sharp edge digging into the stopper. The rounded tip 608 may also guide the needles 600A, 600B into the previously pierced location, thereby reducing stopper fragmentation. Additionally, the nozzle-shaped interior cross-section of needles 600A, 600B can be configured to control the flow characteristics of fluid out of needles 600A, 600B during dispensing. For example, the nozzle-shaped interior cross-section at the tip of needles 600A, 600B can be configured to control the flow rate, speed, direction, and/or pressure of the fluid being dispensed.

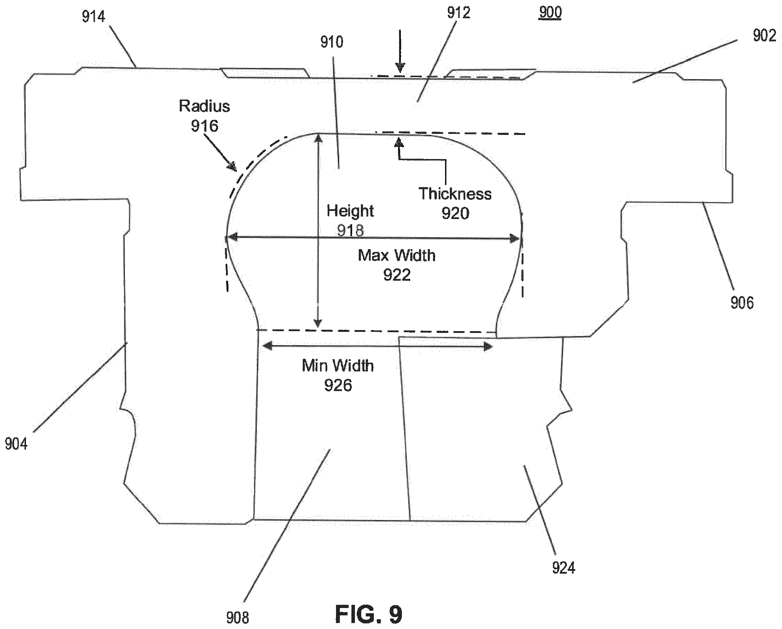

Referring now to FIG. 9, a stopper 900 for a container is provided. In some embodiments, stopper 900 may comprise a lyophilization stopper. Stopper 900 may comprise a plug portion 904 configured to seal an opening of a container, such as containers 106 of FIG. 1A, containers 308A, 308B, 308C of FIG. 3, and containers 408A, 408B of FIGS. 4A and 5B, or other conventional containers or commercially available containers with a crimp-style neck such as vials manufactured by OMPI, Schott, or Wheaton. Plug portion 904 may comprise a diameter that fits any of these containers, e.g., in a range between about 5 mm to about 25 mm. Stopper 900 may also comprise a disk portion 902 disposed on top of the plug portion 904. The disk portion 902 may have a diameter larger than a diameter of an opening of the containers or the diameter of the plug portion 904. For example, the disk portion can have a diameter in a range between about 10 mm to about 32 mm or between about 10 mm to about 20 mm. The disk portion 902 may comprise a flange 906 extending radially beyond an outer diameter of the plug portion 904. The flange 906 may have a thickness in a range between about 1 mm to about 5 mm, e.g., about 1 mm to about 3 mm. In some embodiments, stopper 900 may have a total height in a range of about 5 mm to about 20 mm, e.g., about 10 mm to about 15 mm.

In some embodiments, stopper 900 may comprise a hollow cavity 908 extending through the plug portion 904 and into the disk portion 902. The hollow cavity 908, the plug portion 904, and the disk portion 902 may be positioned coaxial relative to each other. In some embodiments, the disk portion 902 may comprise a pierceable membrane 912 disposed over the proximal end of the hollow cavity 908, and the pierceable membrane 912 may be coaxial with the hollow cavity 908. As illustrated in FIG. 9, in some embodiments, the pierceable membrane 912 may have a thickness 920 that is less than a thickness of the flange 906 such that a probe or a needle can pierce the pierceable membrane 912 of stopper 900 while reducing stopper fragmentation. For example, as discussed in further detail below, a combination of the reduced thickness 920 of pierceable membrane 912 and a bulb-shaped internal cross-section of stopper 900 may allow reduced stopper fragmentation even after repeated penetration of stopper 900. For example, the bulb-shaped interior cross-section of stopper 900 and the reduced thickness 920 at the pierceable membrane 912 may create an integral hinge-like mechanism with a low contact area interface between the needle and stopper 900 along the direction of the needle penetration compared to a thick membrane. The low contact area interface can reduce friction between the needle and the membrane and can allow the piercable membrane 912 to repeatedly "open" without creating substantial fragmentation. By way of example, the pierceable membrane 912 may have a thickness in a range of about 0.5 mm to about 3.0 mm, e.g., about 0.5 mm to about 2 mm. In some embodiments, stopper 900 may be manufactured using an elastomeric rubber material, such as butyl, molded silicone, natural rubber, isoprene, fluorocarbon-based fluroelastomer materials (FKM), or any combination thereof. In some embodiments, stopper 900 may be manufactured using bromobutyl rubber, chlorobutyl rubber, or molded halogenated butyl (or halobutyl) rubber. Halogenated butyl can include bromobutyl and chlorobutyl, and the use of bromobutyl in manufacturing stopper 900 may further support reducing stopper fragmentation.

In some embodiments, stopper 900 may comprise an integral hinge means for enabling repeated penetration of the stopper using, for example, an aspiration needle or probe without causing stopper fragmentation. Such integral hinge means may comprise a molding undercut in the body of stopper 900. For example, as shown in FIG. 9, the hollow cavity 908 of stopper 900 may have a bulb-shaped interior cross-section 910 at a proximal end of the hollow cavity 908. Accordingly, the pierceable membrane 912 of stopper 900 may curve into a sidewall of the proximal end of the hollow cavity 908 so as to form the molding undercut having the bulb-shaped interior cross-section 910. A combination of the reduced thickness 920 of pierceable membrane 912 and the bulb-shaped internal cross-section 910 of stopper 900 may allow reduced stopper fragmentation even after repeated penetration of stopper 900. As discussed above, the bulb-shaped interior cross-section of stopper 900 and the reduced thickness 920 at the pierceable membrane 912 may create an integral hinge-like mechanism with a low contact area interface between the needle and stopper 900 along the direction of the needle penetration compared to a thick membrane. The low contact area interface can reduce friction between the needle and the membrane and can allow the pierceable membrane 912 to repeatedly "open" without creating substantial fragmentation. In some embodiments, a minimum radius of curvature 916 of a transition between the pierceable membrane 912 and a sidewall of the proximal end of hollow cavity 908 may be in a range of about 0.5 mm to about 3.5 mm, e.g., about 2 mm to about 2.5 mm. In other embodiments, a ratio of a maximum width 922 of the bulb-shaped interior cross-section 910 to a minimum interior width 926 of the hollow cavity 908 may be in a range between about 1 to about 3, e.g., about 1 to about 2. For example, the ratio of the maximum width 922 of the bulb-shaped interior cross-section 910 to the minimum interior width 926 of the hollow cavity 908 may be in a range between about 1.1 to about 1.5. In some embodiments, a height 918 of the bulb-shaped interior cross-section may be between about 2 mm and about 10 mm, e.g., about 3 mm to about 8 mm, or about 5 mm to about 5.3 mm.

In some embodiments, stopper 900 may comprise a rectangular cutout 924 on at least one portion of a sidewall of the plug portion 904. The rectangular cutout 924 may have a height in a range of about 3 mm to about 10 mm and a width in a range of about 0.5 mm to about 10 mm. In other embodiments, stopper 900 may comprise one or more protrusions 914 at a top surface of the disk portion 902. For example, stopper 900 may comprise a plurality of protrusions 914 spaced apart from each other at the top surface of the disk portion 902. In some embodiments, the plurality of protrusions 914 may not overlap the pierceable membrane 912.

As discussed above, the molding undercut of stopper 900 having a bulb-shaped interior cross-section 910 may facilitate a large number of repeated penetrations of stopper 900 by, for example, an automated aspiration needle or probe system while minimizing stopper fragmentation. The bulb-shaped interior cross-section 910 and the reduced thickness at the pierceable membrane 912, for example, may reduce contact areas between the needle or probe and stopper 900. Furthermore, the bulb-shaped interior cross-section 910 may increase the radius of the pierceable membrane 912, while ensuring that plug portion 904 remains thick enough to seal the container. The protrusion of cross-section 910 into disk portion 902 can reduce the thickness of pierceable membrane 912, while ensuring that flange 960 remains thick enough to seal the container. For example, the flange 960 may comprise a rubber viscoelastic material that may flow into any surface voids in the container when compressed. Flange 960 may need to be compressed a certain percentage to maintain a suitable seal, imposing a minimum thickness requirement on flange 960. Absent the bulb-shaped interior cross-section 910, the minimum thickness of the flange 960 could constrain the thickness of the pierceable membrane 912. The increased radius and decreased thickness afforded by the integral hinge-like mechanism can support gradual deformation of the pierceable membrane 912 when contacted by the needle or probe. Accordingly, when the needle or probe penetrates stopper 900 at the pierceable membrane 912, the bulb-shaped interior cross-section 910 and the reduced thickness at the pierceable membrane 912 may create an integral hinge-like mechanism with a low contact area interface between the needle and stopper 900 along the direction of the needle penetration compared to a thick membrane. The low contact area interface can reduce friction between the needle and the membrane and can allow the piercable membrane 921 to repeatedly "open" without creating substantial fragmentation. The integral hinge-like mechanism afforded by the bulb-shaped interior cross-section 910 and the reduced thickness at the pierceable membrane 912 may allow the pierceable membrane 912 to flex when the needle or probe pushes on the pierceable membrane 912. As the pierceable membrane 912 flexes downward, an already-pierced hole on stopper 900 may open, thereby reducing the friction between the probe and stopper 900 as the probe slides through the hole. The reduction of friction may, thus, support reduction in stopper fragmentation. In conventional, commercially available stoppers for lyophilized products, the internal geometry is usually tapered or rectangular with straight sidewalls. The internal geometry in conventional stoppers does not support the flexing enabled by the integral hinge-like mechanism in stopper 900. Because stopper 900 may be repeatedly penetrated (e.g., about 10 to about 1000 repeated penetrations) rather than penetrated only once or twice, the integral hinge-like mechanism afforded by the bulb-shaped interior cross-section 910 and the reduced thickness at the pierceable membrane 912 may support a reduction in stopper fragmentation.

The ratio of the maximum width 922 to the minimum width 926, the pierceable membrane thickness 920, and the radius 916 can be tuned to reduce fragmentation of the stopper. Particularly for aspiration needles or probes with a rounded tip, such as rounded tip 510 of FIGS. 5A and 5B, and rounded tip 608 of FIG. 6C, or other conventional or commercially available probes such as probes manufactured by Hamilton Company and Becton Dickinson, the integral hinge-like mechanism created by the bulb-shaped interior cross-section 910 and the reduced thickness of the pierceable membrane 912 relative to flange 906 of stopper 900 may allow repeated penetration of stopper 900, such as tens to hundreds of penetrations of stopper 900, without stopper fragmentation. Accordingly, the container can store a large amount of materials, e.g., reagent materials, for use with many tests, and the materials can be retrieved repeatedly.

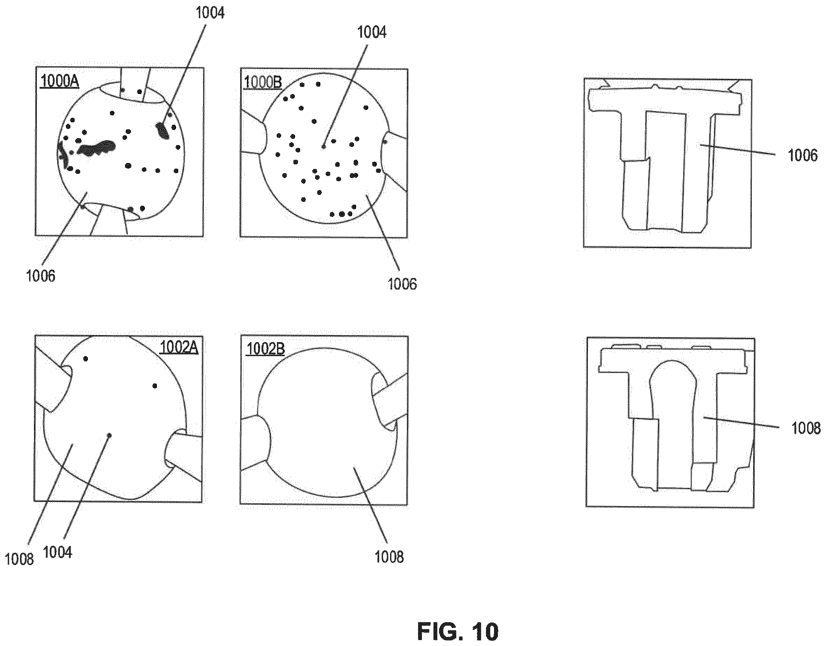

By way of example, FIG. 10 illustrates a comparison of stopper fragmentation in two different stoppers. Images 1000A, 1000B, 1002A, 1002B depict filters from pierce testing, showing aspirated debris caught in a filter. As seen in FIG. 10, images 1000A, 1000B illustrate images from two trials of resultant stopper fragments 1004 produced after repeated penetration of a standard, conventional lyophilization stopper (such as stopper 1006) by a probe having a rounded tip. The conventional stopper, such as stopper 1006, may have a rectangular cross-section, while stopper 1008 consistent with the embodiments of the present disclosure may have a bulb-shaped internal cross-section. On the other hand, images 1002A, 1002B illustrate images from two trials of resultant stopper fragments 1004 produced after about 10 to about 1000, e.g., about 200 to about 500, repeated penetrations by the same probe having a rounded tip of a stopper similar to stopper 900 of FIG. 9 (such as stopper 1008), consistent with the embodiments of the present disclosure. As illustrated in FIG. 10, the integral hinge-like mechanism created by the molding undercut of stopper 1008, including the bulb-shaped interior cross-section and the reduced thickness of the pierceable membrane, may significantly aid in reducing stopper fragmentation even after repeated penetrations of a stopper 1008. For example, as shown in FIG. 10, the number of resultant stopper fragments 1004 is significantly less after repeated penetration of stopper 1008 than after repeated penetration of stopper 1006.

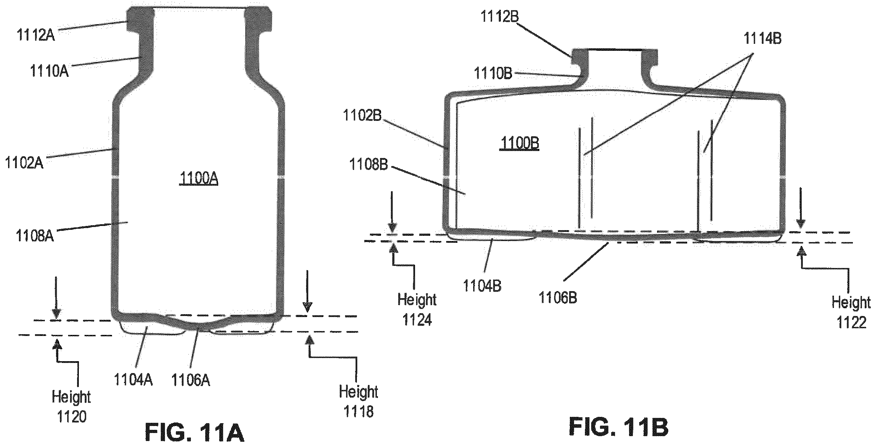

Referring now to FIGS. 11A and 11B, exemplary containers 1100A, 1100B for storing and packaging materials, such as reagent materials, are provided. In some embodiments, containers 1100A, 1100B may comprise molded plastic vials configured to hold fluid material therein. For example, containers 1100A, 1100B may be configured to hold a volume of fluid in a range between about 1 ml and about 100 ml, e.g., 2 ml, 3 ml, 5 ml, 10 ml, 20 ml, 30 ml, 50 ml, or 80 ml. In other embodiments, containers 1100A, 1100B may comprise glass containers configured to hold freeze-dried materials (e.g., lyophilized materials, or the like). Container 1100A may comprise a base 1102A comprising a hollow cavity 1108A. Container 1100A may also comprise a cylindrical structure including a neck portion 1110A extending vertically upward from the base 1102A and a top portion 1112A extending vertically upward from the neck portion 1110A. The cylindrical structure may form an opening and may extend vertically upward from the base 1102A. The outer dimension of the base 1102A may be greater than an outer diameter of the cylindrical structure, including the neck portion 1110A and the top portion 1112A. In some embodiments, an outer diameter of the top portion 1112A may be greater than an outer diameter of the neck portion 1110A. In some embodiments, the base 1102A may comprise a bottom surface with a convex center 1106A protruding from the bottom surface in a direction away from the hollow cavity 1108A such that the convex center 1106A may be configured to pool material inside the container, such as reagent material, towards the convex center 1106A (e.g., a molded fluid-centering design). In some embodiments, the convex center 1106A may protrude in a range of about 1 mm to about 3 mm in height 1118 from the bottom surface of the base 1102A in the direction away from the hollow cavity 1108A. Accordingly, material will pool towards the convex center 1106A of container 1100A, minimizing dead volume and enabling an aspiration needle or probe to aspirate more material from container 1100A than from a flat-bottomed container of equivalent volume.

In some embodiments, container 1100A may also comprise one or more protrusions 1104A disposed on an exterior bottom surface of the base 1102A. In some embodiments, the one or more protrusions 1104A may comprise flat bottom surfaces, and the one or more protrusions 1104A may be configured to substantially surround the convex center 1106A. In some embodiments, a maximum height 1120 of the protrusions 1104A may be equal to or greater than a height 1118 of the convex center 1106A such that when the base 1102A is lying flat on a surface, the protrusions 1104A contacting the surface may provide stability to the container. In some implementations, when the height 1120 is greater than the height 1118, the convex center 1106A may not contact the surface when the container is placed on a flat surface. In some embodiments, a maximum height of the protrusions may equal the height of convex center 1106A, such that when the base 1102A is lying flat on a surface both the protrusions 1104A and the convex center 1106A contact the surface. Accordingly, the one or more protrusions 1104A may allow container 1100A to remain stable on a flat surface, even with the convex center 1106A protruding from the bottom surface of the base 1102A.

In some embodiments, the container may be oblong in shape, rather than cylindrical like container 1100A. For example, container 1100B may have an oblong shape. Container 1100B may comprise a base 1102B comprising a hollow cavity 1108B. Container 1100B may also comprise a cylindrical structure including a neck portion 1110B extending vertically upward from the base 1102B and a top portion 1112B extending vertically upward from the neck portion 1110B. The cylindrical structure may form an opening and may extend vertically upward from the base 1102B. The outer dimension of the base 1102B (e.g., a major axis of base 1102B) may be greater than an outer diameter of the cylindrical structure, including the neck portion 1110B and the top portion 1112B. In some embodiments, the base 1102B may comprise a molded fluid-centering design: a bottom surface with a convex center 1106B protruding from the bottom surface in a direction away from the hollow cavity 1108B such that the convex center 1106B may be configured to pool material inside the container, such as reagent material, towards the convex center 1106B. In some embodiments, the convex center 1106B may protrude in a range of about 1 mm to about 3 mm in height 1122 from the bottom surface of the base 1102B in the direction away from the hollow cavity 1108B. Accordingly, material will pool towards the convex center 1106B of container 1100B, minimizing dead volume and enabling an aspiration needle or probe to aspirate more material from container 1100B than from a flat-bottomed container of equivalent volume.

In some embodiments, the base 1102B of container 1100B may be oblong in shape such that an outer dimension of the base 1102B is a major axis of the oblong. In some embodiments, the base 1102B may comprise one or more indentations 1114B along a first sidewall and one or more indentations 1114B along a second sidewall (not shown) opposite the first sidewall. In some embodiments, container 1100B may also comprise one or more protrusions 1104B disposed on an exterior bottom surface of the base 1102B. In some embodiments, the one or more protrusions 1104B may comprise flat bottom surfaces, and the one or more protrusions 1104B may be configured to substantially surround the convex center 1106B. In some embodiments, a maximum height of the protrusions 1104B may be greater than a height of the convex center 1106B such that when the base 1102B is lying flat on a surface, the protrusions 1104B contact the surface and prevent the convex center 1106B from contacting the surface. In some embodiments, a maximum height 1124 of the protrusions 1104B may equal to or greater than the height 1122 of convex center 1106B, such that when the base 1102B is lying flat on a surface, the protrusions 1104B contacting the surface may provide stability to the container. In some implementations, when the height 1124 is greater than the height 1122, the convex center 1106B may not contact the surface when the container is placed on a flat surface. Accordingly, the one or more protrusions 1104B may allow container 1100B to remain stable on a flat surface even with the convex center 1106B protruding from the bottom surface of the base 1102B.

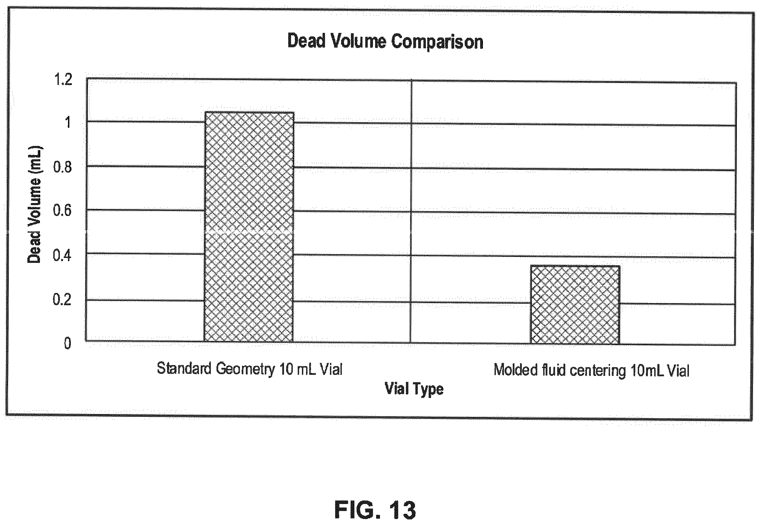

FIGS. 12 and 13 illustrate dead volume comparisons between a conventional container 1200 and container 1100A of FIG. 11A, consistent with the embodiments of the present disclosure. As shown in FIG. 12, a conventional container 1200 comprises a base 1202 comprising a hollow cavity 1208. Container 1200 also comprises a cylindrical structure including a neck portion 1210 extending vertically upward from the base 1202 and a top portion 1212 extending vertically upward from the neck portion 1210. However, unlike the base 1102A of container 1100A, the base 1202 of container 1200 comprises a bottom surface with an inverted geometry 1218 protruding towards the hollow cavity 1208. Due to the inverted geometry 1218 of the base 1202, material contained in container 1200 will pool away from the center of the bottom surface. In addition, when probe 1116 accesses the material contained in container 1200, any material left remaining in container 1200 will be below the bottom reach of the probe 1116 and, thus, the remaining material (the dead volume) will be lost and thrown out. On the other hand, when probe 1116 accesses the material contained in container 1100A, the material contained therein may pool toward the convex center 1106A and, thus, probe 1116 may be able to aspirate more of the remaining material, thereby reducing the dead volume. For example, as shown in FIG. 13, a bar graph comparing the dead volume in conventional standard geometry containers (such as container 1200 in FIG. 12) and the dead volume in molded fluid-centering containers (such as container 1100A in FIG. 12) is shown. As illustrated in FIG. 13, dead volume is significantly reduced by about 70% with molded fluid-centering containers, such as container 1100A in FIG. 12, because the convex center 1106A may be capable of pooling material towards the center of the container, thereby enabling a probe, such as probe 1116, to aspirate the material. Accordingly, container 1100A may allow more material to be aspirated and reduce total costs that are spent on obtaining the material. Moreover and optionally, container 1100A may be manufactured using plastic (such as high density poly ethylene (HDPE) or other plastic materials) while conventional container 1200 may be manufactured using glass. Because plastic is a hydrophobic by nature, the material of container 1100A may allow fluid within container 1100A to flow all the way to the bottom without wicking up the sides of the container 1100A.

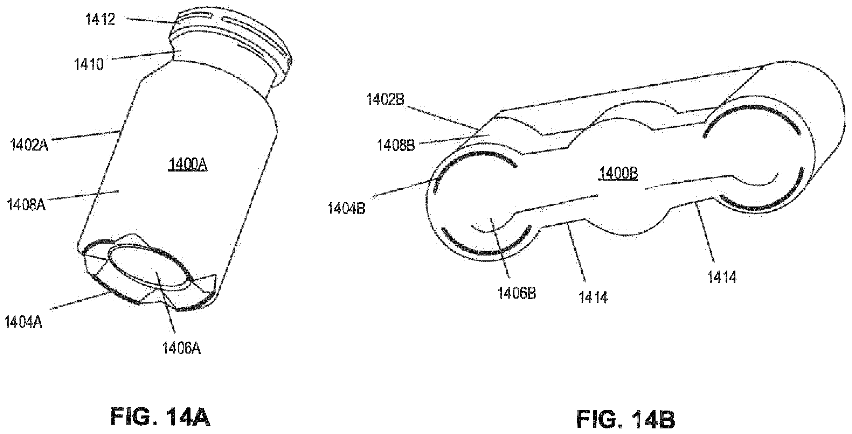

Referring now to FIGS. 14A and 14B, exemplary containers 1400A, 1400B for storing and packaging materials, such as reagent materials, are provided. In some embodiments, containers 1400A, 1400B may comprise molded plastic vials configured to hold fluid material therein. For example, containers 1400A, 1400B may be configured to hold a volume of fluid in a range between about 1 ml and about 100 ml, e.g., 2 ml, 3 ml, 5 ml, 10 ml, 20 ml, 30 ml, 50 ml, or 80 ml. Container 1400A may comprise a base 1402A comprising a hollow cavity 1408A. Container 1400A may also comprise a cylindrical structure including a neck portion 1410 extending vertically upward from the base 1402A and a top portion 1412 extending vertically upward from the neck portion 1410. The cylindrical structure may form an opening and may extend vertically upward from the base 1402A. The outer dimension of the base 1402A (e.g., a major axis of base 1402A) may be greater than an outer diameter of the cylindrical structure, including the neck portion 1410 and the top portion 1412. In some embodiments, an outer diameter of the top portion 1412 may be greater than an outer diameter of the neck portion 1410. In some embodiments, the base 1402A may comprise a bottom surface with a convex center 1406A protruding from the bottom surface in a direction away from the hollow cavity 1408A such that the convex center 1406A may be configured to pool material, inside the container such as reagent material, towards the convex center 1406A. In some embodiments, the convex center 1406A may protrude in a range of about 1 mm to about 3 mm in height from the bottom surface of the base 1402A in the direction away from the hollow cavity 1408A, similar to the convex center 1106 in FIG. 11A. Accordingly, material will pool towards the convex center 1406A of container 1400A, minimizing dead volume and enabling an aspiration needle or probe to aspirate more material from container 1400A than from a flat-bottomed container of equivalent volume.

In some embodiments, container 1400A may also comprise one or more protrusions 1404A disposed on an exterior bottom surface of the base 1402A. The one or more protrusions may comprise, for example, one or more ridges positioned around the convex center 1406A. For example, the one or more protrusions 1404A may be configured to substantially surround the convex center 1406A. In some embodiments, a maximum height of the protrusions 1404A may be greater than a height of the convex center 1406A such that when the base 1402A is lying flat on a surface, the protrusions 1404A contact the surface and the convex center 1406A does not contact the surface. In some embodiments, a maximum height of the protrusions 1404A may equal to the height of convex center 1406A, such that when the base 1402A is lying flat on a surface, both the protrusions 1404A and the convex center 1406A contact the surface. Accordingly, the one or more protrusions 1404A may allow container 1400A to remain stable on a flat surface even with the convex center 1406A protruding from the bottom surface of the base 1402A.

In some embodiments, the container may be oblong in shape, rather than cylindrical like container 1400A. For example, container 1400B may have an oblong shape. Container 1400B may comprise a base 1402B comprising a hollow cavity 1408B. Container 1400B may also comprise a cylindrical structure including a neck portion (similar to neck portion 1110B in FIG. 11B) extending vertically upward from the base 1402B and a top portion (similar to top portion 1112B in FIG. 11B) extending vertically upward from the neck portion. The cylindrical structure may form an opening and may extend vertically upward from the base 1402B. The outer dimension of the base 1402B (e.g., a major axis of base 1402B) may be greater than an outer diameter of the cylindrical structure, including the neck portion and the top portion. In some embodiments, the base 1402B may comprise a bottom surface with a convex center 1406B protruding from the bottom surface in a direction away from the hollow cavity 1408B such that the convex center 1406B may be configured to pool material inside the container, such as reagent material, towards the convex center 1406B. In some embodiments, the convex center 1406B may protrude in a range of about 1 mm to about 3 mm in height from the bottom surface of the base 1402B in the direction away from the hollow cavity 1408B. Accordingly, material will pool towards the convex center 1406B of container 1400B, minimizing dead volume and enabling an aspiration needle or probe to aspirate more material from container 1400B than from a flat-bottomed container of equivalent volume.

In some embodiments, the base 1402B of container 1400B may be oblong in shape such that an outer dimension of the base 1402B is a major axis of the oblong. In some embodiments, the base 1402B may comprise one or more indentations 1414 along a first sidewall and one or more indentations 1414 along a second sidewall opposite the first sidewall. In some embodiments, container 1400B may also comprise one or more protrusions 1404B disposed on an exterior bottom surface of the base 1402B. In some embodiments, the one or more protrusions 1404B may comprise one or more ridges positioned around the convex center 1406B. For example, the one or more protrusions 1404B may be configured to substantially surround the convex center 1406B. In some embodiments, a maximum height of the protrusions 1404B may be equal to or greater than a height of the convex center 1406B such that when the base 1402B is lying flat on a surface, the protrusions 1404B contacting the surface may provide stability to the container. In some implementations, when the height of the protrusions 1404B is greater than the height of the convex center 1406B, the convex center 1406B may not contact the surface when the container 1400B is placed on a flat surface. Accordingly, the one or more protrusions 1404B may allow container 1400B to remain stable on a flat surface even with the convex center 1406B protruding from the bottom surface of the base 1402B. While FIGS. 14A and 14B illustrate cylindrical and oblong containers, the embodiments of the present disclosure are not limited to the illustrated cylindrical or oblong containers. For example, various shapes of containers may be placed and stored in the cartridge assemblies of the present disclosure, including but not limited to rectangular, triangular, or polygonal containers.

## Claims

1. A probe assembly (110) for piercing a container (106; 308A; 308B; 308C; 408A; 408B; 812; 1100A; 1100B; 1400A; 1400B), comprising:
a first needle (502) comprising a hollow cavity (504) and a pointed tip (506) at a proximal end of the first needle (502); and
a second needle (508) disposed inside the hollow cavity (504) of the first needle (502), the second needle (508) comprising a rounded tip (510; 608) at a proximal end of the second needle (508),
wherein the first needle (502) and the second needle (508) are positioned coaxial relative to each other,
wherein the first needle (502) and the second needle (508) are independently actuatable,
wherein the first needle (502) is configured to move from a first position to a second position when actuated, and
wherein the second needle (508) is configured to move from a third position to a fourth position when actuated,
**characterised in that**
the second needle (508) comprises a second hollow cavity and is capable of aspirating material through the second hollow cavity,
wherein, when actuated, one of the first needle (502) and the second needle (508) is configured to move to puncture a stopper (120; 704; 804; 900; 1008) of a container (106; 308A; 308B; 308C; 408A; 408B; 812; 1100A; 1100B; 1400A; 1400B).

2. The probe assembly (110) of claim 1, wherein the first needle (502) is configured to pierce a thick stopper (120, 704, 900, 1008) and the second needle (508) is configured to puncture a thin stopper (804) or previously pierced location on a stopper.

3. The probe assembly (110) of claim 1 or 2, wherein the second needle (508) comprises a plurality of stepped sections (602A, 604A, 606A; 602B, 604B) having different diameters.

4. The probe assembly (110) of claim 3, wherein the diameter of the sections (602A, 604A, 606A; 602B, 604B) increases from the section (606A; 604B) that is configured to first penetrate the stopper (120; 704; 804; 900; 1008) to the near end section (602A; 602B),
wherein each section (602A, 604A, 606A; 602B, 604B) optionally has a constant diameter.

5. The probe assembly (110) of any one of the claims 1 to 4, wherein the second needle (508) comprises a nozzle-shaped interior cross-section at the tip of the second needle (508).

6. A testing system comprising a cartridge assembly (100) and a probe system (500) configured to access a container (106; 308A; 308B; 308C; 408A; 408B; 812; 1100A; 1100B; 1400A; 1400B) packaged in the cartridge assembly (100), the cartridge assembly (100) comprising:
a first housing (102) comprising a first set of cavities, the first set of cavities being configured to accommodate one or more containers;
a second housing (104) configured to be coupled to the first housing (102), the second housing (104) comprising a second set of cavities, wherein the second set of cavities corresponds to the first set of cavities when the second housing (104) is coupled to the first housing (102); and
wherein the cartridge assembly (100) comprises, for each cavity of the second housing (104), means for aligning the cartridge assembly (100) and a container (106; 308A; 308B; 308C; 408A; 408B; 812; 1100A; 1100B; 1400A; 1400B) accommodated in the cavity with a mating component at a proximal end of the probe system (500),
the probe system (500) comprising a probe assembly according to claim 1.

7. The testing system of claim 6, wherein the first needle (502) is configured to pierce a thick stopper (120, 704, 900, 1008) and the second needle (508) is configured to puncture a thin stopper (804), or previously pierced location on a stopper

8. The testing system of claim 6 or 7, wherein the means for aligning the cartridge assembly (100) and the container (106; 308A; 308B; 308C; 408A; 408B; 812; 1100A; 1100B; 1400A; 1400B) accommodated in the cavity comprise a means for securing the container (106; 308A; 308B; 308C; 408A; 408B; 812; 1100A; 1100B; 1400A; 1400B) within the cartridge assembly (100) when the second housing (104) is coupled to the first housing (102).

9. The testing system of any one of the claims 6 to 8, wherein the first housing (102) comprises, for each cavity of the first housing (102), means for aligning the container (106; 308A; 308B; 308C; 408A; 408B; 812; 1100A; 1100B; 1400A; 1400B) accommodated in the cavity with a center of the cavity, and/or
wherein a plurality of the first set of cavities and corresponding cavities of the second set of cavities are configured to accommodate a single container (106; 308A; 308B; 308C; 408A; 408B; 812; 1100A; 1100B; 1400A; 1400B) or a plurality of containers when the first housing (102) is coupled to the second housing (104).

10. The testing system of any one of the claims 6 to 9, wherein the plurality of the first set of cavities and the corresponding cavities of the second set of cavities are configured to accommodate a single container (106; 308A; 308B; 308C; 408A; 408B; 812; 1100A; 1100B; 1400A; 1400B), the single container (106; 308A; 308B; 308C; 408A; 408B; 812; 1100A; 1100B; 1400A; 1400B) being oblong in shape, and/or
a volume of the single container (106; 308A; 308B; 308C; 408A; 408B; 812; 1100A; 1100B; 1400A; 1400B) being between 4 ml and 60 ml.

11. The testing system of any one of the claims 6 to 10,
wherein the second housing (104) comprises a plurality of alignment features (108) as the means for aligning corresponding to the first set of cavities and the second set of cavities,
wherein each of the plurality of alignment features (108) is positioned coaxial to a center of a corresponding cavity of the first set of cavities and the second set of cavities, and
wherein each of the plurality of alignment features (108) is configured to mate with a component at a proximal end of the probe system (500).

12. The testing system of claim 11, wherein the plurality of alignment features (108) comprises a plurality of openings on a top surface of the second housing (104), wherein each of the plurality of openings comprises a plurality of protrusions positioned circumferentially around each of the plurality of openings,
wherein preferably the plurality of protrusions are biased vertically downward such that the plurality of protrusions are configured to apply a force vertically downward on top of the one or more containers accommodated in the first housing (102) and the second housing (104).

13. The testing system of claim 11 or 12, wherein the plurality of alignment features (108) are configured to align the cartridge assembly (100) with the probe system (500) when the plurality of alignment features (108) mate with the component at the proximal end of the probe system (500).

14. The testing system of any one of the claims 6 to 13, wherein the container (1100A) accommodated in the cavity comprises:
a base (1102A) comprising a hollow cavity (1108A); and
a cylindrical structure forming an opening and extending vertically upward from the base (1102A),
wherein an outer dimension of the base (1102A) is greater than an outer diameter of the cylindrical structure, and
wherein the base (1102A) comprises a bottom surface with a convex center (1106A) protruding from the bottom surface in a direction away from the hollow cavity (1108A), the convex center (1106A) being configured to pool fluid in the container towards the convex center (1106A).

15. A testing method for accessing a container (106; 308A; 308B; 308C; 408A; 408B; 812; 1100A; 1100B; 1400A; 1400B) packaged in a cartridge assembly (100) with a probe system (500),
the probe system (500) comprising a probe assembly according to claim 1.

## Patentansprüche

1. Eine Sondenanordnung (110) zum Durchstechen eines Behälters (106; 308A; 308B; 308C; 408A; 408B; 812; 1100A; 1100B; 1400A; 1400B), umfassend:
eine erste Nadel (502) mit einer hohlen Kavität (504) und einer spitzen Spitze (506) an einem proximalen Ende der ersten Nadel (502); und
eine zweite Nadel (508), die innerhalb der hohlen Kavität (504) der ersten Nadel (502) angeordnet ist, wobei die zweite Nadel (508) eine abgerundete Spitze (510; 608) an einem proximalen Ende der zweiten Nadel (508) aufweist,
wobei die erste Nadel (502) und die zweite Nadel (508) koaxial zueinander positioniert sind,
wobei die erste Nadel (502) und die zweite Nadel (508) unabhängig voneinander betätigbar sind,
wobei die erste Nadel (502) konfiguriert ist, um sich bei Betätigung von einer ersten Position in eine zweite Position zu bewegen, und
wobei die zweite Nadel (508) konfiguriert ist, um sie sich bei Betätigung von einer dritten Position in eine vierte Position zu bewegen,
**dadurch gekennzeichnet, dass**
die zweite Nadel (508) eine zweite hohle Kavität umfasst und in der Lage ist, Material durch die zweite hohle Kavität anzusaugen,
wobei bei Betätigung eine von der ersten Nadel (502) und der zweiten Nadel (508) dazu konfiguriert ist, sich zu bewegen, um einen Stopfen (120; 704; 804; 900; 1008) eines Behälters (106; 308A; 308B; 308C; 408A; 408B; 812; 1100A; 1100B; 1400A; 1400B) zu durchstechen.

2. Die Sondenanordnung (110) nach Anspruch 1, wobei die erste Nadel (502) dazu konfiguriert ist, einen dicken Stopfen (120, 704, 900, 1008) zu durchstechen, und die zweite Nadel (508) dazu konfiguriert ist, einen dünnen Stopfen (804) oder eine zuvor durchstochene Stelle an einem Stopfen zu durchstechen.

3. Die Sondenanordnung (110) nach Anspruch 1 oder 2, wobei die zweite Nadel (508) mehrere abgestufte Abschnitte (602A, 604A, 606A; 602B, 604B) mit unterschiedlichen Durchmessern umfasst.

4. Die Sondenanordnung (110) nach Anspruch 3, wobei der Durchmesser der Abschnitte (602A, 604A, 606A; 602B, 604B) ausgehend vom Abschnitt (606A; 604B), der konfiguriert ist, um den Stopfen (120; 704; 804; 900; 1008) zuerst zu durchstechen, bis zum nahen Endabschnitt (602A; 602B) zunimmt,
wobei jeder Abschnitt (602A, 604A, 606A; 602B, 604B) optional einen konstanten Durchmesser aufweist.

5. Die Sondenanordnung (110) nach irgendeinem der Ansprüche von 1 bis 4, wobei die zweite Nadel (508) einen düsenförmigen Innenquerschnitt an der Spitze der zweiten Nadel (508) aufweist.

6. Ein Testsystem, das eine Kartuschenanordnung (100) und ein Sondensystem (500) umfasst, das konfiguriert ist, um auf einen Behälter (106; 308A; 308B; 308C; 408A; 408B; 812; 1100A; 1100B; 1400A; 1400B) zuzugreifen, der in der Kartuschenanordnung (100) verpackt ist, wobei die Kartuschenanordnung (100) Folgendes umfasst:
ein erstes Gehäuse (102) mit einem ersten Satz von Kavitäten, wobei der erste Satz von Kavitäten konfiguriert ist, um einen oder mehrere Behälter aufzunehmen;
ein zweites Gehäuse (104), das konfiguriert ist, um mit dem ersten Gehäuse (102) verbunden zu werden, wobei das zweite Gehäuse (104) einen zweiten Satz von Kavitäten umfasst, wobei der zweite Satz von Kavitäten dem ersten Satz von Kavitäten entspricht, wenn das zweite Gehäuse (104) mit dem ersten Gehäuse (102) gekoppelt ist; und
wobei die Kartuschenanordnung (100) für jede Kavität des zweiten Gehäuses (104) Mittel zum Ausrichten der Kartuschenanordnung (100) und eines in der Kavität aufgenommenen Behälters (106; 308A; 308B; 308C; 408A; 408B; 812; 1100A; 1100B; 1400A; 1400B) mit einem passenden bzw. Gegenbauteil (*mating component*) an einem proximalen Ende des Sondensystems (500),
wobei das Sondensystem (500) eine Sondenanordnung nach Anspruch 1 umfasst.

7. Das Testsystem nach Anspruch 6, wobei die erste Nadel (502) konfiguriert ist, um einen dicken Stopfen (120, 704, 900, 1008) zu durchstechen, und die zweite Nadel (508) konfiguriert ist, um einen dünnen Stopfen (804) oder eine zuvor durchstochene Stelle an einem Stopfen zu durchstechen.

8. Das Testsystem nach Anspruch 6 oder 7, wobei die Mittel zum Ausrichten der Kartuschenanordnung (100) und des in der Kavität aufgenommenen Behälters (106; 308A; 308B; 308C; 408A; 408B; 812; 1100A; 1100B; 1400A; 1400B) ein Mittel zum Sichern des Behälters (106; 308A; 308B; 308C; 408A; 408B; 812; 1100A; 1100B; 1400A; 1400B) innerhalb der Kartuschenanordnung (100) umfasst, wenn das zweite Gehäuse (104) mit dem ersten Gehäuse (102) gekoppelt ist.

9. Das Testsystem nach irgendeinem der Ansprüche von 6 bis 8, wobei das erste Gehäuse (102) für jede Kavität des ersten Gehäuses (102) Mittel zum Ausrichten des in der Kavität aufgenommenen Behälters (106; 308A; 308B; 308C; 408A; 408B; 812; 1100A; 1100B; 1400A; 1400B) mit einer Mitte der Kavität, und/oder
wobei eine Vielzahl aus dem ersten Satz von Kavitäten und entsprechende Kavitäten des zweiten Satzes von Kavitäten konfiguriert sind, um einen einzelnen Behälter (106; 308A; 308B; 308C; 408A; 408B; 812; 1100A; 1100B; 1400A; 1400B) oder eine Vielzahl von Behältern aufzunehmen, wenn das erste Gehäuse (102) mit dem zweiten Gehäuse (104) gekoppelt ist.

10. Das Testsystem nach irgendeinem der Ansprüche von 6 bis 9, wobei die Vielzahl aus dem ersten Satz von Kavitäten und die entsprechenden Kavitäten des zweiten Satzes konfiguriert sind, um einen einzelnen Behälter (106; 308A; 308B; 308C; 408A; 408B; 812; 1100A; 1100B; 1400A; 1400B) aufzunehmen, wobei der einzelne Behälter (106; 308A; 308B; 308C; 408A; 408B; 812; 1100A; 1100B; 1400A; 1400B) eine längliche Form aufweist, und/oder wobei
ein Volumen des einzelnen Behälters (106; 308A; 308B; 308C; 408A; 408B; 812; 1100A; 1100B; 1400A; 1400B) zwischen 4 ml und 60 ml liegt.

11. Das Testsystem nach irgendeinem der Ansprüche von 6 bis 10,
wobei das zweite Gehäuse (104) eine Vielzahl von Ausrichtungsmerkmalen (108) als Mittel zum Ausrichten entsprechend dem ersten Satz von Kavitäten und dem zweiten Satz von Kavitäten umfasst,
wobei jedes Merkmal aus der Vielzahl von Ausrichtungsmerkmalen (108) koaxial zu einer Mitte einer entsprechenden Kavität des ersten Satzes von Kavitäten und des zweiten Satzes von Kavitäten positioniert ist, und
wobei jedes Merkmal aus der Vielzahl von Ausrichtungsmerkmalen (108) konfiguriert ist, um mit einer Komponente an einem proximalen Ende des Sondensystems (500) zusammenzupassen bzw. in Eingriff zu kommen.

12. Das Testsystem nach Anspruch 11, wobei die Vielzahl von Ausrichtungsmerkmalen (108) eine Vielzahl von Öffnungen auf einer oberen Oberfläche des zweiten Gehäuses (104) umfasst, wobei jede Öffnung der Vielzahl von Öffnungen eine Vielzahl von Vorsprüngen umfasst, die entlang des Umfangs um jede Öffnung der Vielzahl von Öffnungen herum angeordnet sind,
wobei vorzugsweise die Vielzahl von Vorsprüngen vertikal nach unten vorgespannt sind, so dass die Vielzahl von Vorsprüngen so konfiguriert sind, dass sie eine Kraft vertikal nach unten auf die obere Oberfläche des einen oder der mehreren Behälter ausüben, die im ersten Gehäuse (102) und dem zweiten Gehäuse (104) aufgenommen sind.

13. Das Testsystem nach Anspruch 11 oder 12, wobei die Vielzahl von Ausrichtungsmerkmalen (108) konfiguriert sind, um die Kartuschenanordnung (100) mit dem Sondensystem (500) auszurichten, wenn die Vielzahl von Ausrichtungsmerkmalen (108) mit der Komponente am proximalen Ende des Sondensystems (500) in Eingriff kommen.

14. Das Testsystem nach irgendeinem der Ansprüche von 6 bis 13, wobei der in der Kavität aufgenommene Behälter (1100A) Folgendes umfasst:
eine Basis (1102A), die eine hohle Kavität (1108A) umfasst; und
eine zylindrische Struktur, die eine Öffnung bildet und sich vertikal von der Basis (1102A) nach oben erstreckt,
wobei eine Außenabmessung der Basis (1102A) größer ist als ein Außendurchmesser der zylindrischen Struktur, und
wobei die Basis (1102A) eine Bodenfläche mit einem konvexen Zentrum (1106A) umfasst, die von der Bodenfläche in einer Richtung weg von der hohlen Kavität (1108A) vorsteht, wobei das konvexe Zentrum (1106A) konfiguriert ist, um Flüssigkeit im Behälter zum konvexen Zentrum (1106A) hin zu sammeln.

15. Ein Testverfahren für den Zugriff auf einen in einer Kartuschenanordnung (100) verpackten Behälter (106; 308A; 308B; 308C; 408A; 408B; 812; 1100A; 1100B; 1400A; 1400B) mit einem Sondensystem (500),
wobei das Sondensystem (500) eine Sondenanordnung nach Anspruch 1 umfasst.

## Revendications

1. Un ensemble de sonde (110) destiné à percer un récipient (106 ; 308A ; 308B ; 308C ; 408A ; 408B ; 812 ; 1100A ; 1100B ; 1400A ; 1400B), comprenant :
une première aiguille (502) comprenant une cavité creuse (504) et une pointe acérée (506) à une extrémité proximale de la première aiguille (502) ; et
une deuxième aiguille (508) agencée à l'intérieur de la cavité creuse (504) de la première aiguille (502), la deuxième aiguille (508) comprenant une pointe arrondie (510 ; 608) à une extrémité proximale de la deuxième aiguille (508),
sachant que la première aiguille (502) et la deuxième aiguille (508) sont positionnées de manière coaxiale l'une par rapport à l'autre,
sachant que la première aiguille (502) et la deuxième aiguille (508) peuvent être actionnées indépendamment,
sachant que la première aiguille (502) est configurée pour se déplacer d'une première position à une deuxième position lorsqu'elle est actionnée, et
sachant que la deuxième aiguille (508) est configurée pour se déplacer d'une troisième position à une quatrième position lorsqu'elle est actionnée,
**caractérisé en ce que**
la deuxième aiguille (508) comprend une deuxième cavité creuse et est apte à aspirer un matériau à travers la deuxième cavité creuse,
sachant que, lorsqu'elle est actionnée, l'une parmi la première aiguille (502) et la deuxième aiguille (508) est configurée pour se déplacer afin de percer un bouchon (120 ; 704 ; 804 ; 900 ; 1008) d'un récipient (106 ; 308A ; 308B ; 308C ; 408A ; 408B ; 812 ; 1100A ; 1100B ; 1400A ; 1400B).

2. L'ensemble de sonde (110) d'après la revendication 1, sachant que la première aiguille (502) est configurée pour percer un bouchon épais (120, 704, 900, 1008) et que la deuxième aiguille (508) est configurée pour piquer ou encore perforer (*puncture*) un bouchon mince (804) ou un emplacement préalablement percé sur un bouchon.

3. L'ensemble de sonde (110) d'après la revendication 1 ou 2, sachant que la deuxième aiguille (508) comprend une pluralité de sections étagées (602A, 604A, 606A ; 602B, 604B) ayant des diamètres différents.

4. L'ensemble de sonde (110) d'après la revendication 3, sachant que le diamètre des sections (602A, 604A, 606A ; 602B, 604B) augmente depuis la section (606A ; 604B) qui est configurée pour pénétrer en premier dans le bouchon (120 ; 704 ; 804 ; 900 ; 1008) à la section d'extrémité proche (602A ; 602B),
sachant que chaque section (602A, 604A, 606A ; 602B, 604B) présente facultativement un diamètre constant.

5. L'ensemble de sonde (110) d'après l'une quelconque des revendications de 1 à 4, sachant que la deuxième aiguille (508) comprend une section transversale intérieure en forme de buse à la pointe de la deuxième aiguille (508).

6. Un système de test comprenant un ensemble de cartouche (100) et un système de sondes (500) configuré pour accéder à un récipient (106 ; 308A ; 308B ; 308C ; 408A ; 408B ; 812 ; 1100A ; 1100B ; 1400A ; 1400B) contenu (*packaged*) dans l'ensemble de cartouche (100), l'ensemble de cartouche (100) comprenant :
un premier boîtier (102) comprenant un premier ensemble de cavités, le premier ensemble de cavités étant configuré pour loger un ou plusieurs récipients ;
un deuxième boîtier (104) configuré pour être couplé au premier boîtier (102), le deuxième boîtier (104) comprenant un deuxième ensemble de cavités, sachant que le deuxième ensemble de cavités correspond au premier ensemble de cavités lorsque le deuxième boîtier (104) est couplé au premier boîtier (102) ; et
sachant que l'ensemble de cartouche (100) comprend, pour chaque cavité du deuxième boîtier (104), des moyens pour aligner l'ensemble de cartouche (100) et un récipient (106 ; 308A ; 308B ; 308C ; 408A ; 408B ; 812 ; 1100A ; 1100B ; 1400A ; 1400B) logé dans la cavité avec un composant d'accouplement de contrepartie (*mating component*) à une extrémité proximale du système de sonde (500),
le système de sonde (500) comprenant un ensemble de sonde d'après la revendication 1.

7. Le système de test d'après la revendication 6, sachant que la première aiguille (502) est configurée pour percer un bouchon épais (120, 704, 900, 1008) et que la deuxième aiguille (508) est configurée pour piquer ou encore perforer (*puncture*) un bouchon mince (804) ou un emplacement préalablement percé sur un bouchon.

8. Le système de test d'après la revendication 6 ou 7, sachant que les moyens pour l'alignement de l'ensemble de cartouche (100) et du récipient (106 ; 308A ; 308B ; 308C ; 408A ; 408B; 812 ; 1100A ; 1100B ; 1400A ; 1400B) logé dans la cavité comprennent un moyen pour fixer le récipient (106 ; 308A ; 308B ; 308C ; 408A ; 408B ; 812 ; 1100A ; 1100B ; 1400A ; 1400B) à l'intérieur de l'ensemble de cartouche (100) lorsque le deuxième boîtier (104) est couplé au premier boîtier (102).

9. Le système de test d'après l'une quelconque des revendications de 6 à 8, sachant que le premier boîtier (102) comprend, pour chaque cavité du premier boîtier (102), des moyens pour aligner le récipient (106 ; 308A ; 308B ; 308C ; 408A ; 408B ; 812 ; 1100A ; 1100B ; 1400A ; 1400B) logé dans la cavité, avec le centre de la cavité, et/ou
sachant qu'une pluralité du premier ensemble de cavités et des cavités correspondantes du deuxième ensemble de cavités sont configurées pour loger un seul récipient (106 ; 308A ; 308B ; 308C ; 408A ; 408B ; 812 ; 1100A ; 1100B ; 1400A ; 1400B) ou une pluralité de récipients lorsque le premier boîtier (102) est couplé au deuxième boîtier (104).

10. Le système de test d'après l'une quelconque des revendications de 6 à 9, sachant que la pluralité du premier ensemble de cavités et les cavités correspondantes du deuxième ensemble de cavités sont configurées pour loger un seul récipient (106 ; 308A ; 308B ; 308C ; 408A ; 408B ; 812 ; 1100A ; 1100B ; 1400A ; 1400B), le seul récipient (106 ; 308A ; 308B ; 308C ; 408A ; 408B ; 812 ; 1100A ; 1100B ; 1400A ; 1400B) étant de forme oblongue, et/ou
un volume du seul récipient (106 ; 308A ; 308B ; 308C ; 408A ; 408B ; 812 ; 1100A ; 1100B ; 1400A ; 1400B) étant compris entre 4 ml et 60 ml.

11. Le système de test d'après l'une quelconque des revendications de 6 à 10,
sachant que le deuxième boîtier (104) comprend une pluralité de caractéristiques d'alignement (108) servant de moyens d'alignement correspondant au premier ensemble de cavités et au deuxième ensemble de cavités,
sachant que chacune de la pluralité de caractéristiques d'alignement (108) est positionnée de manière coaxiale par rapport au centre d'une cavité correspondante du premier ensemble de cavités et du deuxième ensemble de cavités, et
sachant que chacune de la pluralité de caractéristiques d'alignement (108) est configurée pour s'accoupler avec un composant à une extrémité proximale du système de sonde (500).

12. Le système de test d'après la revendication 11, sachant que la pluralité de caractéristiques d'alignement (108) comprend une pluralité d'ouvertures sur une surface supérieure du deuxième boîtier (104), sachant que chacune de la pluralité d'ouvertures comprend une pluralité de saillies positionnées de manière circonférentielle autour de chacune de la pluralité d'ouvertures,
sachant que, de préférence, la pluralité de saillies est inclinée verticalement vers le bas, de manière que la pluralité de saillies soit configurée pour appliquer une force verticalement vers le bas sur le dessus d'un ou plusieurs récipients logés dans le premier boîtier (102) et le deuxième boîtier (104).

13. Le système de test d'après la revendication 11 ou 12, sachant que la pluralité de caractéristiques d'alignement (108) est configurée pour aligner l'ensemble de cartouche (100) avec le système de sonde (500) lorsque la pluralité de caractéristiques d'alignement (108) s'accouple avec le composant à l'extrémité proximale du système de sonde (500).

14. Le système de test d'après l'une quelconque des revendications de 6 à 13, sachant que le récipient (1100A) logé dans la cavité comprend :
une base (1102A) comprenant une cavité creuse (1108A) ; et
une structure cylindrique formant une ouverture et s'étendant verticalement vers le haut à partir de la base (1102A),
sachant qu'une dimension extérieure de la base (1102A) est supérieure à un diamètre extérieur de la structure cylindrique, et
sachant que la base (1102A) comprend une surface inférieure avec un centre convexe (1106A) faisant saillie depuis la surface inférieure dans une direction s'éloignant de la cavité creuse (1108A), le centre convexe (1106A) étant configuré pour accumuler le fluide dans le récipient vers le centre convexe (1106A).

15. Un procédé de test pour accéder à un récipient (106 ; 308A ; 308B ; 308C ; 408A ; 408B ; 812 ; 1100A ; 1100B ; 1400A ; 1400B) contenu (*packaged*) dans un ensemble de cartouche (100), à l'aide d'un système de sonde (500),
le système de sonde (500) comprenant un ensemble de sonde d'après la revendication 1.
